Europäisches Patentamt

European Patent Office

Office européen des brevets

(19)

(11) Numéro de publication: **0 349 353**
**A1**

# DEMANDE DE BREVET EUROPEEN

(12)

(21) Numéro de dépôt: 89400813.5

(22) Date de dépôt: 22.03.89

(51) Int. Cl.⁵: **C 12 N 15/00**
C 12 N 1/20, C 07 K 13/00
//(C12N1/20,C12R1:125)

(30) Priorité: 22.03.88 FR 8803736
02.06.88 FR 8807379
05.08.88 FR 8810647

(43) Date de publication de la demande:
03.01.90 Bulletin 90/01

(84) Etats contractants désignés:
AT BE CH DE ES FR GB GR IT LI LU NL SE

(71) Demandeur: INSTITUT PASTEUR
28, rue du Docteur Roux
F-75724 Paris Cédex 15 (FR)

(72) Inventeur: Kunst, Frederik
46, rue Barbès (B18)
F-94200 Ivry (FR)

Debarbouille, Michel
53b ld Arago
F-75013 Paris (FR)

Msadek, Tarek
18, rue Violet
F-75015 Paris (FR)

Rapoport, Georges
20, rue de l'Armorique
F-75015 Paris (FR)

Klier, André
6, Allée Guynemer
F-93330 Neuilly sur Marne (FR)

Dedonder, Raymond
3, Allée des Pépinières
F-92290 Chatenay-Malabry (FR)

(74) Mandataire: Grosset-Fournier, Chantal Catherine et al
SC Ernest Gutmann/Yves Plasseraud 67 boulevard Haussmann
F-75008 Paris (FR)

(54) Séquences d'ADN comprenant la partie fonctionnelle du locus sacU et du locus sacUh de B.SUBTILIS, vecteurs contenant ces séquences, et leur utilisation dans des procédés de production de protéines.

(57) L'invention concerne un fragment d'acide nucléique contenant une séquence fonctionnelle de nucléotides contenue dans un fragment de 2,55 kb issu du chromosome d'une souche de B. subtilis, ou contenue dans un fragment de 14 kb issu du chromosome d'une souche de B.subtilis présentant une mutation sacUh, ladite séquence étant caractérisée en ce qu'elle est capable, lorsqu'elle est introduite dans un B. subtilis- ou dans un autre micro-organisme, capable d'exploiter à son profit l'information portée à ce fragment- d'y induire une surproduction de protéines ou de lui conférer un phénotype Degh.

L'invention concerne également un procédé de stimulation de la production d'un polypeptide déterminé par un micro-organisme approprié par introduction du susdit fragment d'ADN dans les cellules du micro-organisme dont le génome ou a été modifié de manière à ce qu'il contienne une séquence d'ADN codant pour ledit polypeptide déterminé dont le promoteur est précédé d'une séquence cible.

EP 0 349 353 A1

## Description

### SEQUENCES D'ADN COMPRENANT LA PARTIE FONCTIONNELLE DU LOCUS sacU et du locus sacUh DE B. SUBTILIS, VECTEURS CONTENANT CES SEQUENCES, ET LEUR UTILISATION DANS DES PROCEDES DE PRODUCTION DE PROTEINES

La présente invention concerne des séquences d'ADN comprenant une partie fonctionnelle du locus sacU et du locus sacUh situés sur le génome de B. subtilis, ainsi que les vecteurs contenant cette séquence, et l'application de ces séquences et des vecteurs à la stimulation de la production de protéines par B. subtilis ou tout autre micro-organisme approprié dans lesquels ils auront au préalable été incorporés.

Il a déjà été fait référence, sous la désignation "gène sacU", à un gène de régulation de l'expression de gènes de structure codant pour des enzymes de dégradation chez B. subtilis (LEPESANT et al, Mol. Gen. Genet., 118 : 135-160 (1972) ; KUNST et al, Biochimie 56 : 1481-1489 (1974) ; STEINMETZ et al, Mol. Gen. Genet., 148 : 281-285 (1976) ; ZUKOWSKI et al, Gene, 46 : 247-255 (1986)).

Il existe également chez B. subtilis au moins deux autres gènes de régulation de l'expression d'une classe de gènes de structure codant pour des enzymes dégradatives, le gène prtR (NAGAMI et al, J. Bacteriol., 166 : 20-28 (1986) ; YANG et al, J. Bacteriol., 169 : 434-437 (1987)), et le gène sacQ (LEPESANT et al (1972), précédemment cité, KUNST et al (1974), précédemment cité ; TOMIOKA et al, J. Biotechnol., 3 : 85-96 (1985) ; YANG et al, J. Bacteriol., 166 : 113-119 (1986) ; AMORY et al, J. Bacteriol., 169 : 324-333 (1987)).

Ces trois types de gènes de régulation sont des gènes pléïotropes contrôlant l'expression d'un grand nombre de gènes codant pour la production d'enzymes dégradatives dans diverses souches de Bacillus.

Parmi les enzymes codées par les gènes structuraux sous le contrôle de sacU, sacQ et prtR, et sécrétées par B. subtilis dans le milieu de culture, on distingue principalement la lévane-saccharase, les protéases alcaline et neutre, l'α-amylase, des glycanases (PRIEST, Bacteriol. Rev., 41 : 711-753 (1977)).

Des mutations intervenant au niveau des loci sacU et sacQ du génome de B. subtilis conduisent à l'hyperproduction par ce dernier des enzymes sus-mentionnées.

De telles mutations, désignées par les génotypes sacUh et sacQh, peuvent conférer aux souches de B. subtilis les phénotypes Lvsh, Prth et Amyh se manifestant respectivement par des hyperproductions de lévane-saccharase, de protéases et d'α-amylase.

A l'inverse, un autre type de mutation, mise en évidence uniquement au niveau du locus sacU, réduit considérablement les taux de synthèse de lévane-saccharase et de protéase chez B. subtilis. Cette dernière mutation confère aux souches de B. subtilis les phénotypes Lvs- et Prt-, et est désignée par le génotype sacU-.

En résumé, les loci sacU et sacQ seront décrits par sacU+, sacQ+ indiquant le niveau de synthèse de la souche de B. subtilis Marburg 168 de référence et ses dérivés, ou par les génotypes mutés sacUh, sacQh indiquant un niveau de synthèse augmenté ou par sacU- indiquant un niveau de synthèse considérablement réduit.

Au niveau du locus sacU, les mutations sacU- et sacUh sont étroitement liées (6 à 9 % de recombinaison par transformation) et sont donc soit portées par le même gène soit portées par deux gènes étroitement liés (STEINMETZ et al, Mol. Gen. Genet., 148 : 281-285, (1976)).

Les loci sacU et sacQ semblent agir de manière dépendante. Le phénotype de l'hyperproduction d'enzymes (ou encore phénotype Degh, synthèse d'enzymes dégradatives haut niveau) conféré par la présence, soit d'une mutation sacQh chromosomique, soit de multiples copies de sacQ ou de prtR portées par des plasmides, dans la cellule hôte, est aboli par la présence d'une mutation sacU- (HENNER et al, Proceedings of the fifth international symposium on the genetics of industrial microorganisms, Split, Yugoslavia, pp 81-90, 1986) ; AMORY et al (1987), précédemment cité ; TANAKA et KAWATA, J. Bacteriol. , 170 : 3593-3600 (1988)).

C'est donc en raison de leurs effets, dont on a montré qu'il s'exerçait au niveau transcriptionnel sur la synthèse de différentes enzymes chez Bacillus, que des loci sacU, sacQ, ou autres séquences d'ADN d'activitité comparable, issus de différentes souches de Bacillus suscitent beaucoup d'intérêt, notamment pour leur application dans le domaine de la synthèse d'enzymes particulièrement intéressantes au niveau industriel.

Notamment, plusieurs équipes de recherche ont récemment décrit le clonage de fragments d'ADN, à partir de différentes souches de Bacillus, lesdits fragments permettant l'hyperproduction de protéases, ou d'autres enzymes, chez B. subtilis.

Le brevet japonais déposé le 19/07/1983 par OKADA et al sous le n° 60-30 685 décrit un ADN recombinant contenant une séquence d'insertion originaire du génome d'un B. licheniformis présenté comme codant pour une protéase de ce dernier type de micro-organisme. Les auteurs du brevet ont utilisé cet ADN recombinant pour transformer des cultures de Bacillus subtilis en vue d'accroître leur capacité à produire des protéases alcaline et neutre.

OKADA et al (Appl. Microbiol. Biotechnol., 20 : 406-412, (1984)) ont cloné un fragment EcoRI de 3,6 kb à partir de B. licheniformis qui stimule aussi bien la secrétion de la protéase neutre que celle de la protéase alcaline (70x), mais n'a qu'un faible effet sur les enzymes du type α-amylase (2x) ; le phénotype observé est conservé à l'occasion du clonage d'un fragment PvuII de 1,25 kb et même d'un fragment Sau 3A1 de 0,37 kb obtenu à partir du fragment EcoRI.

TOMIOKA et al (J. Biotechnol.,3 : 85-96; (1985)) ont aussi décrit la stimulation de la production des protéases neutre (10x) et alcaline (9x) avec un fragment EcoRI de 4 kb provenant de B. amyloliquefaciens ; ce fragment a pu être réduit à 1,75 kb comprenant des phases ouvertes de lecture non identifiées ; il ne permet pas la stimulation de la synthèse d'α-amylase.

Un fragment d'ADN issu de Bacillus natto a été décrit comme permettant une hyperproduction de protéases alcaline et neutre et de lévane-saccharase (J. Bacteriol.,166 : 20-28, (1986)). Les auteurs ont envisagé qu'un polypeptide de 60 acides aminés pouvait intervenir dans la stimulation de cette production.

Des polypeptides de 46 acides aminés codés par le gène sacQ de B. subtilis (polypeptide SacQS (J. Bacteriol.,166 : 113-119, (1986), et de B. amylolique faciens (polypeptide SacQA) (J. Biotechnol., 3, 85-96, (1985)) ont été décrits comme étant des activateurs de la synthèse d'enzymes dégradatives.

Le brevet français déposé le 18/08/1986 sous le n° 86.11826 aux noms de l'INSTITUT PASTEUR et du CENTRE NATIONAL DE LA RECHERCHE SCIENTIFIQUE décrit l'identification du gène sacQ de B. licheniformis ainsi que du polypeptide SacQL codé par ledit gène sacQ, et un procédé de stimulation de la production d'un polypeptide déterminé par une souche de B. subtilis dont le génome avait été modifié de manière à ce qu'il comprenne soit de manière intégrée dans le chromosome, soit sur un plasmide, une séquence d'ADN codant pour ledit polypeptide déterminé et à laquelle avait également auparavant été incorporé un fragment d'ADN, co-dant pour SacQL.

Toutefois, les loci sacU et sacUh n'ont jamais été clonés et n'ont, par conséquent, jamais été utilisés pour la mise en oeuvre d'un procédé de surproduction d'enzymes, par réintroduction dans une cellule-hôte capable ou rendue capable de produire ces enzymes, ce procédé utilisant les séquences fonctionnelles de ces loci pour leurs propriétés stimulantes à l'égard de cette production.

L'objectif de la présente invention est le clonage du gène sacU et du gène sacUh afin de permettre l'utilisation de ces loci sacU et sacUh, ou d'une séquence fonctionnelle de ces loci, pour la mise en oeuvre d'un procédé de surproduction d'enzymes ou d'autres protéines par B. subtilis ou par tout autre micro-organisme approprié, avec un taux de surproduction aussi élevé que possible.

En effet, les tentatives de clonage du gène sacU qui ont été antérieurement envisagées, ont échoué. Celle décrite dans l'article d'AUBERT et al paru en 1985 dans J. Bacteriol., 161 : 1182-1187, qui décrivait une tentative de clonage de sacU par l'intermédiaire de plasmides navettes capables de se répliquer à la fois dans Escherichia coli et Bacillus subtilis, a été reniée par les mêmes auteurs, dans un autre article en 1987, dans J. Bacteriol., 169 : 3393, au motif qu'elle était erronée

La stratégie décrite par DEBARBOUILLE et al, dans FEMS Microbiol. Lett., 41 : 137-140 (1987)) et qui a été appliquée avec succès au clonage du gène sacS, n'a pas pu être transposée au clonage et à l'identification de sacU. En particulier, il a été procédé à une tentative d'introduire une banque de gènes (sous forme de plasmides recombinants les contenant et obtenus chez Escherichia coli), dans une souche de B. subtilis dont le génome portait une mutation sacU− ainsi qu'un gène de résistance à la kanamycine sous le contrôle du promoteur du gène sacB codant pour la lévane-saccharase. Sous l'effet de sacU−, le gène sacB et celui codant pour la résistance à la kanamycine sont très faiblement exprimés. Il était espéré qu'un des plasmides recombinants qui pouvait contenir le locus sacU, serait capable de conférer à la souche de B. subtilis sacU−, sus-mentionnée, un haut niveau de résistance à la kanamycine et de restaurer la production de lévane-saccharase chez cette même souche. Mais il n'a pas été possible d'isoler un plasmide comportant le locus sacU de cette manière.

La présente invention découle de l'hypothèse (qui semble s'être vérifiée) que ces divers échecs étaient peut-être dus à une toxicité du locus sacU à l'égard d'E. coli.

Aussi, un des buts de la présente invention a été de proposer une méthode pour le clonage du locus sacU directement chez Bacillus subtilis.

Les caractéristiques essentielles du processus qui a conduit à l'invention seront plus aisément appréhendées à la lumière des dessins dans lesquels :

- la figure 1 est une représentation schématique du plasmide pBU3, avec dans la partie supérieure de la figure, le fragment BamHI de 13,5 kb, issu de B. subtilis BD1238, contenant le transposon Tn917lacZ à proximité du locus sacU, et dans la partie inférieure de la figure, le vecteur plasmidique utilisé pour le clonage de ce fragment BamHI,

- la figure 2 est une représentation schématique du plasmide pBU14 avec dans la partie supérieure de la figure, un fragment SalI de 3,9 kb issu du plasmide pBU3 de la figure 1 contenant le locus sacU, et, dans la partie inférieure de la figure le vecteur plasmidique utilisé pour le clonage de ce fragment SalI.

- la figure 3 représente schématiquement les plasmides pHV1431 et pHV1436 utilisés pour le clonage de sacU,

- la figure 4 représente la séquence complète et la carte de restriction d'un fragment de 2550 paires de bases comprenant le locus sacU, avec l'indication de ses sites de restriction,

- la figure 5 représente la séquence en acides aminés du polypeptide SacUS1 (ou DegS)

- la figure 6 représente la séquence en acides aminés du polypeptide SacUS2 (ou DegU)

- la figure 7 représente la comparaison des parties C-terminales du polypeptide SacUS1 (ou DegS) codé par ORF1 du locus sacU de B. subtilis, et des protéines CheA de S. thyphimurium et CpxA de E. coli,

- la figure 8 représente la comparaison du polypeptide SacUS2 (ou DegU) codé par ORF2 du locus sacU de B. subtilis, et des protéines Dye ; UvrC (codé par l'ORF2), OmpR de E. coli, et SpoOA, SpoOF de B. subtilis,

- la figure 9 représente la comparaison des parties C-terminales du polypeptide SacUS2 de B. subtilis, et des protéines UvrC (codées par ORF1 et ORF2) et MalT de E. coli,

- la figure 10 représente la séquence nucléotidique détaillée du fragment d'ADN contenant le locus sacU, ainsi que les polypeptides SacU$_{S1}$ (DegS) et SacU$_{S2}$ (DegU) représentés respectivement au-dessus de phases de lecture ouverte ORF1 et ORF2,

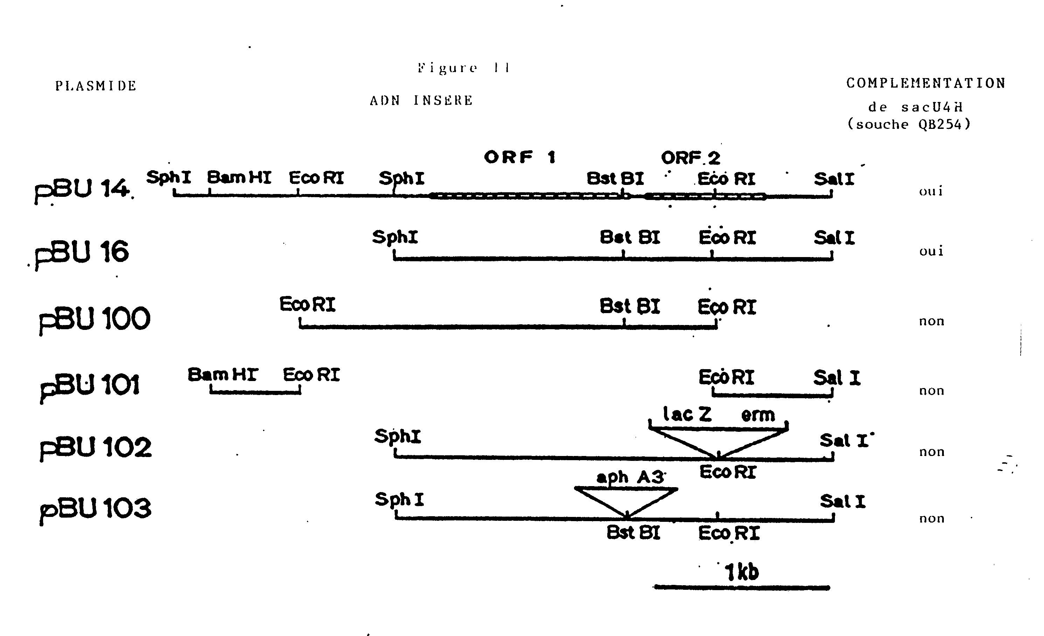

- la figure 11 représente les cartes de restriction de fragments d'ADN issus des fragments de 2,55 kb comprenant le locus sacU et insérés respectivement dans les plasmides pBU14, pBU16, pBU100, pBU101, pBU102 et pBU103,

- la figure 12 représente l'identification des polypeptides SacU$_{S1}$ et SacU$_{S2}$ par migration électrophorétique suivie d'une autoradiographie.

- la figure 13 représente le polypeptide DegS, ainsi que lesq polypeptides DegS220, DegS200, DegS100, et DegS39 (ou DegS42) sécrétés par des souches de B. subtilis portant une mutation sacU$^h$.

- la figure 14 représente le polypeptide DegU, ainsi que les polypeptides DegU32, DegU24 (ou DegU500), DegU146, DegU9 (DegU118), DegU31, DegU143, DegU200, DegU122 et DegU193 sécrétés par des souches de B. subtilis portant une mutation sacU$^h$.

- la figure 15 représente les modifications de nucléotides et d'acides aminés entre différents polypeptides sécrétés par les souches de B. subtilis. sacU$^h$.

Au cours d'une analyse génétique effectuée sur B. subtilis Marburg 168 et ses dérivés (LEPESANT et al, Mol. Gen. Genet., 118 : 135-160 (1972)) il avait été montré par les inventeurs que la région sacU pouvait être située sur le génome à proximité du marqueur hisA1.

Partant de ce résultat, leur attention devait être attirée par la souche de B. subtilis BD1238 (décrite par HAHN et al, J. Bacteriol., 169 : 3104-3109 (1987), et ALBANO et al, J. Bacteriol., 169 : 3110-3117 (1987)) dans laquelle le transposon Tn917lacZ était introduit à proximité du marqueur hisA1.

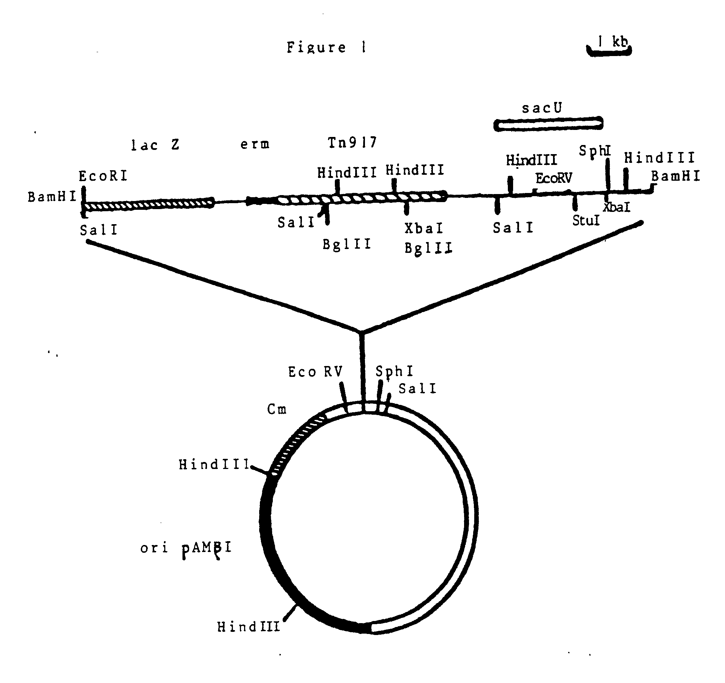

Le génome de B. subtilis BD1238 est en effet caractérisé par la mutation com-524 proche de hisA1, due à l'insertion dans son génome du transposon Tn917lacZ dont la carte de restriction apparait dans la figure 1. Cette région Tn917lacZ comprend le marqueur de résistance à l'érythromycine (PERKINS et al, Proc. Natl. Acad. Sci., USA, 83 : 140-144 (1986) ; SHAW et al, J. Bacteriol., 164 : 782-796 (1985)). Les inventeurs devaient en déduire que chez B. subtilis BD1238 (de génotype sacU$^+$), le locus sacU$^+$ devait être à proximité de la région Tn917lacZ.

Le clonage de la région correspondante du génome de B. subtilis BD1238 en utilisant le marqueur de résistance à l'érythromycine comme moyen de sélection, dans un plasmide capable de se répliquer dans B. subtilis, devait confirmer la validité de cette hypothèse.

La présence d'un site BamHI unique à l'extrémité gauche de Tn917lacZ, a conduit les inventeurs à utiliser l'enzyme de restriction correspondante pour fragmenter le génome de B. subtilis BD1238. Les fragments BamHI obtenus ont été ligaturés dans des plasmides appropriés (figure 1) ; ces derniers ont été utilisés pour transformer des cellules de B. subtilis non résistantes à l'érythromycine, et les cellules de B. subtilis devenues résistantes à l'érythromycine ont été sélectionnées.

Les inventeurs ont ainsi isolé un plasmide (plasmide pBU3) et l'ont utilisé pour transformer des cellules de B. subtilis porteuses d'une mutation sacU$^-$ (phénotype Lvs$^-$) (figure 1).

Ils ont ainsi déterminé que ce plasmide contient un fragment BamHI de 13,5 kb issu de B. subtilis BD1238 capable de restaurer la synthèse de la lévane-saccharase (phénotype Lvs$^+$) chez une souche de B. subtilis portant une mutation sacU$^-$ (phénotype Lvs$^-$).

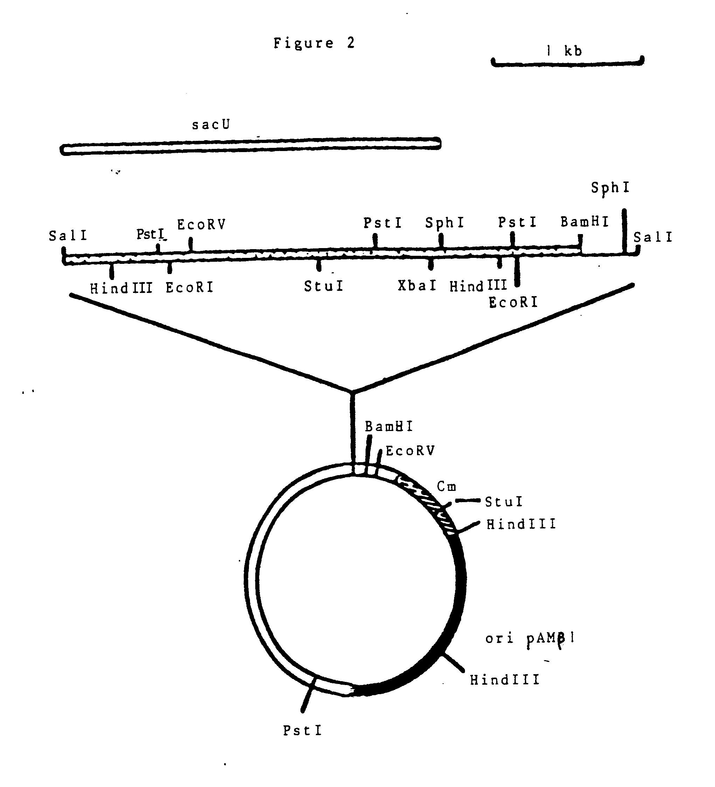

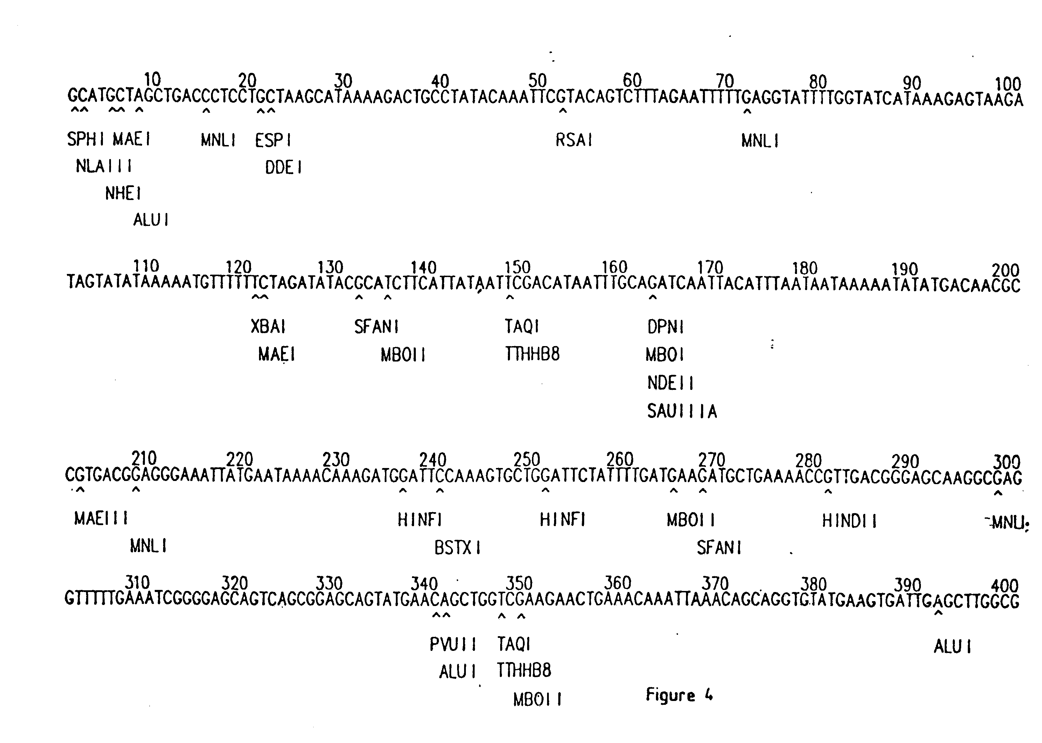

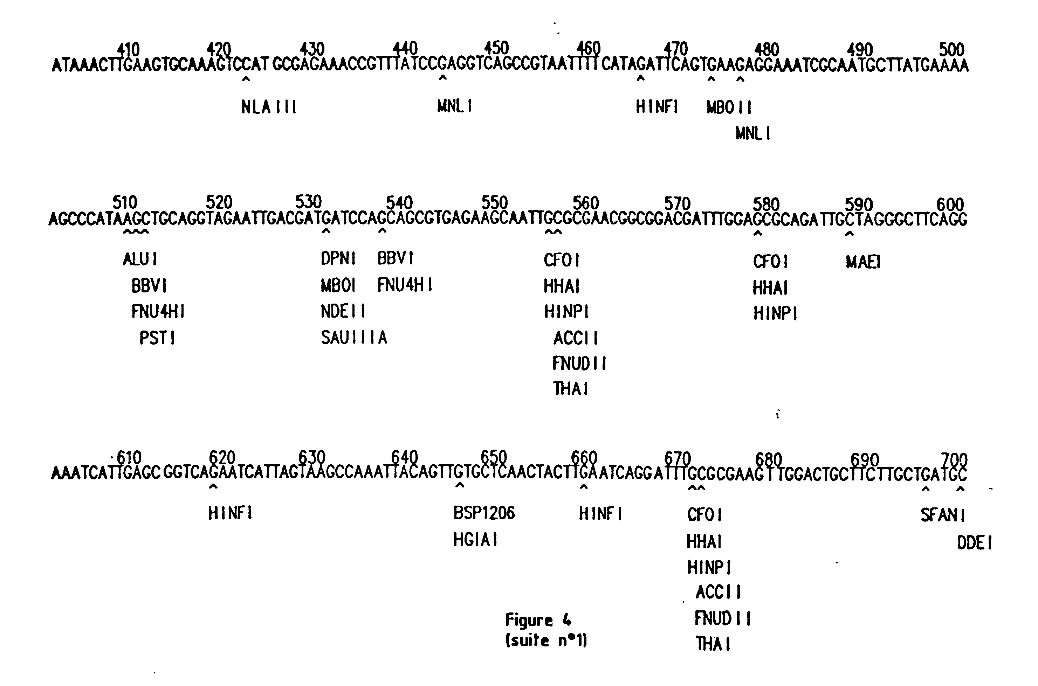

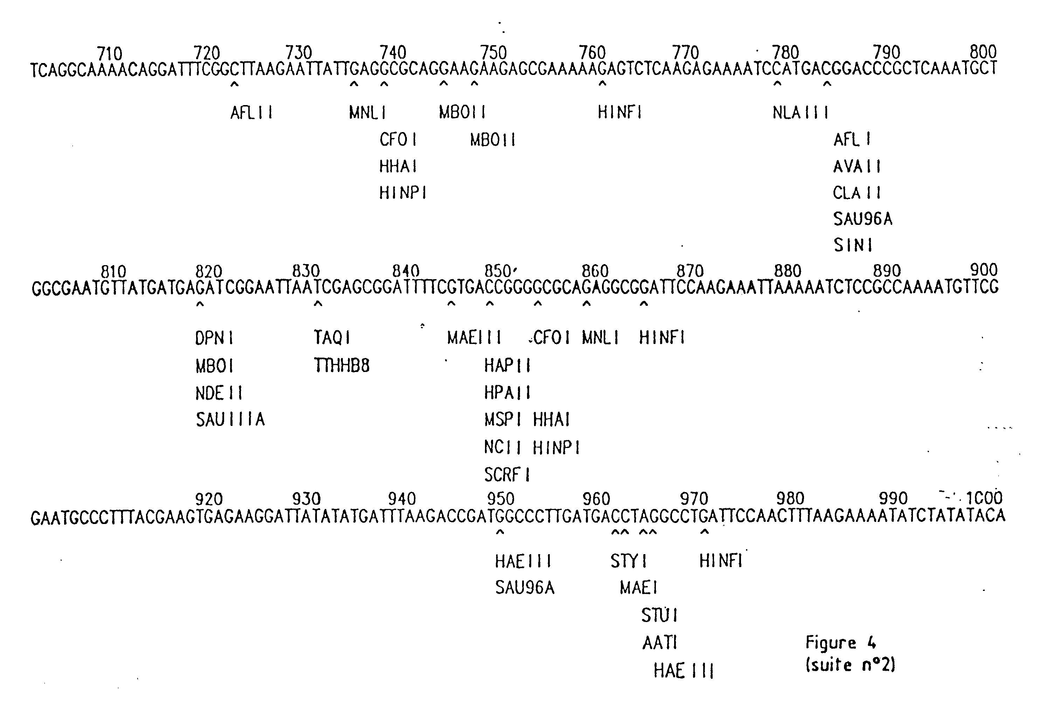

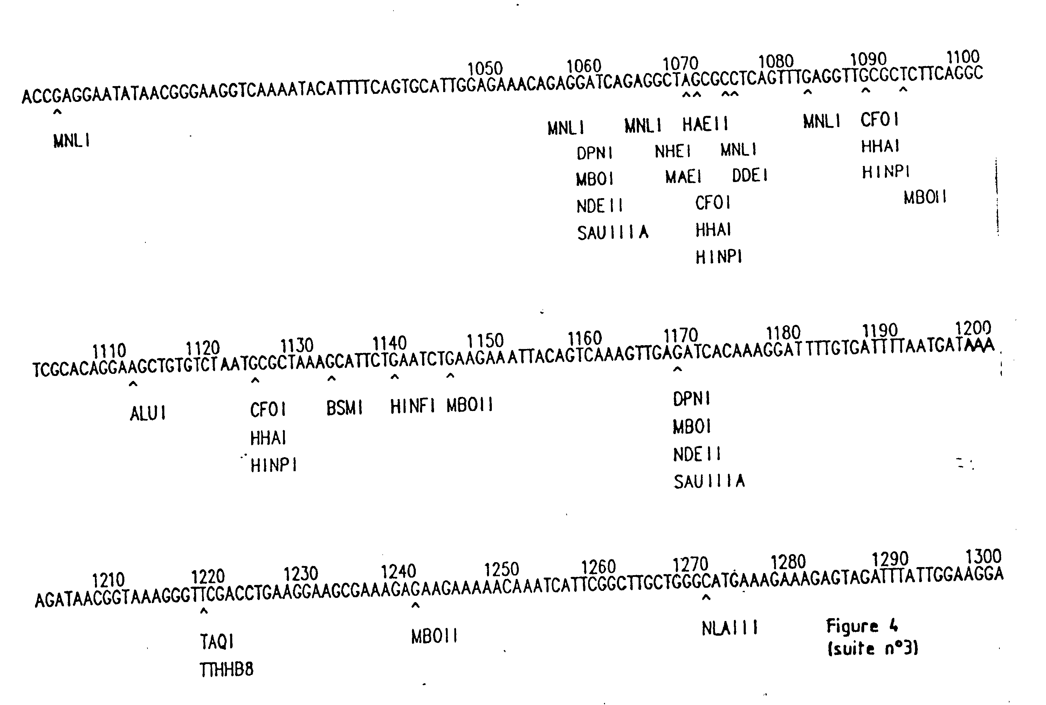

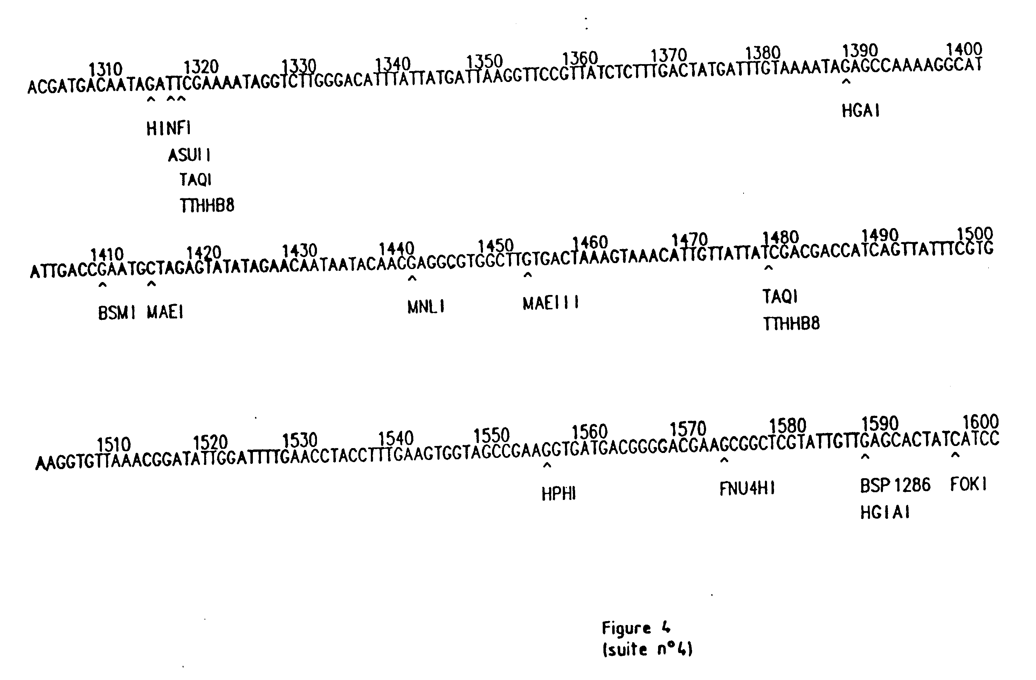

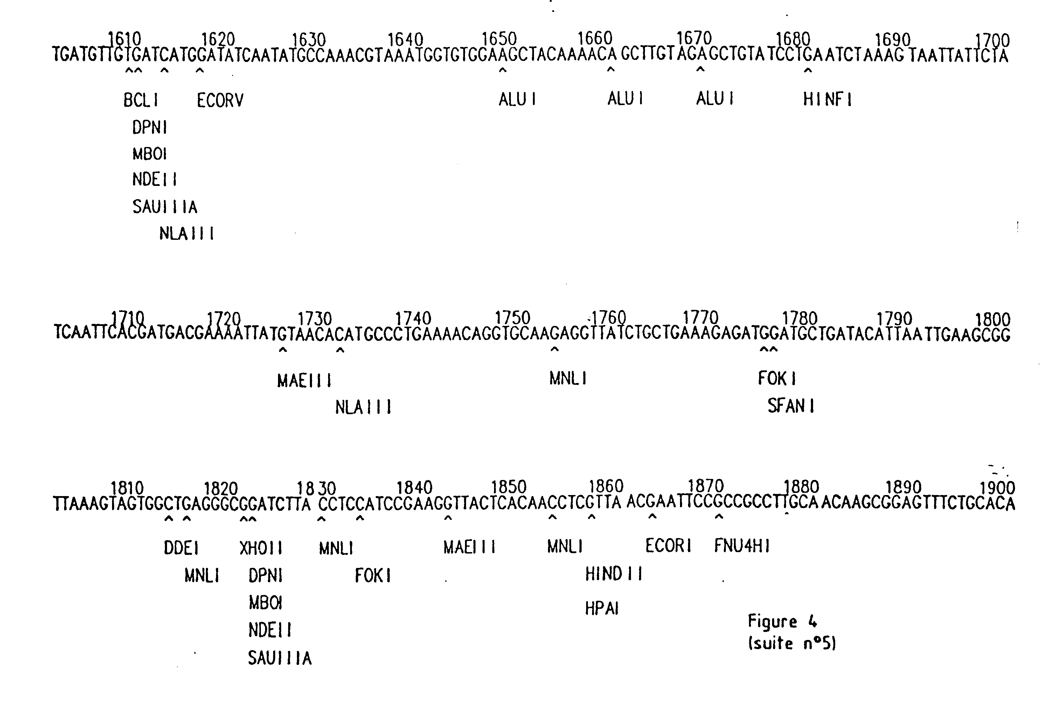

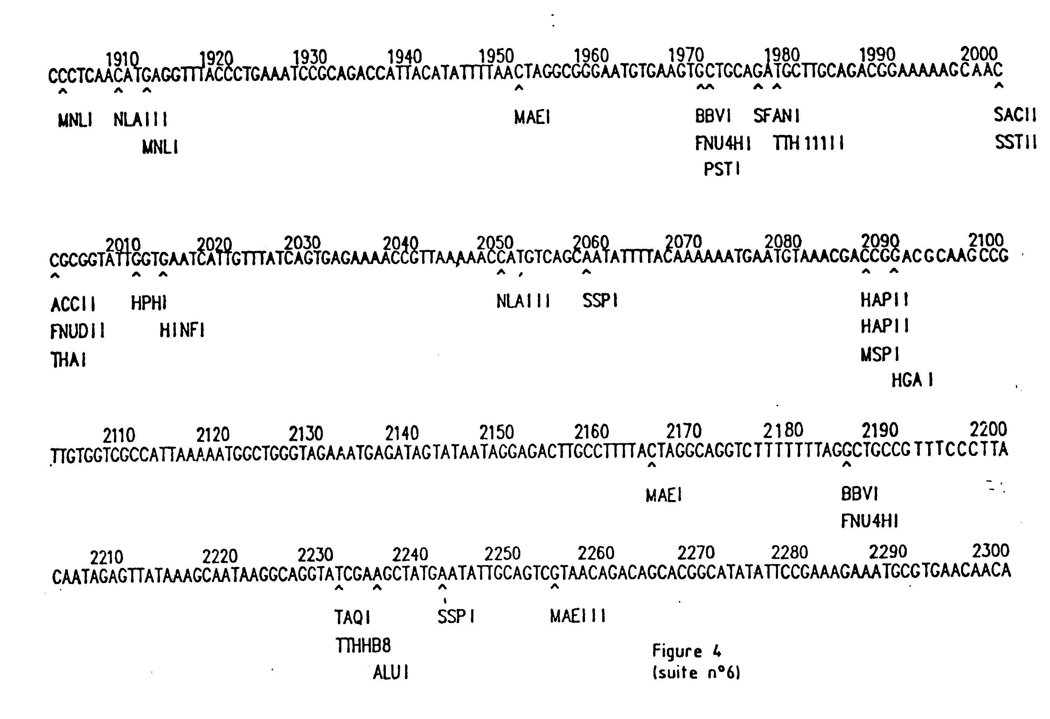

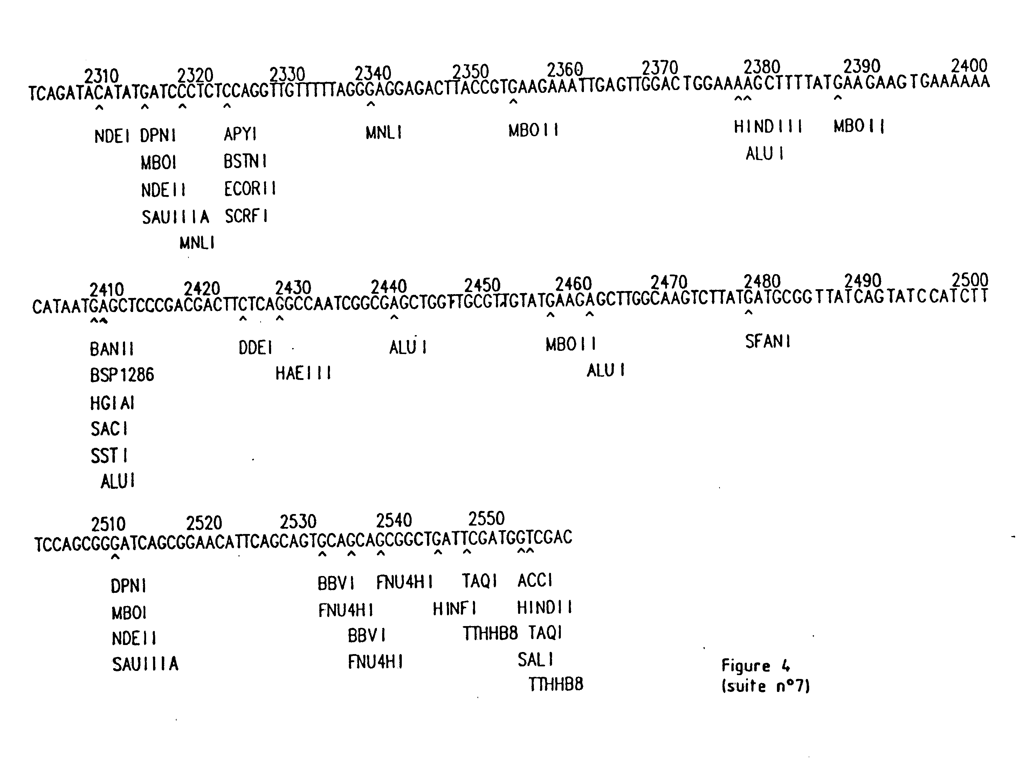

Un premier sous-clonage d'un fragment SalI qui comprend un fragment SalI-BamHI provenant de l'ADN de B.subtilis BD1238 de 3,6 kb et un fragment de 0,28 kb BamHI-SalI de pBR322 donne le plasmide pBU14 (figure 2) qui est aussi capable de restaurer la synthèse de la lévane-saccharase chez un mutant sacU$^-$. L'élimination dans ce fragment de la séquence comprise entre les deux sites EcoRI et la religature conduit à un plasmide qui a perdu toute activité et ne complémente plus une souche sacU$^-$. D'autre part, le clonage du fragment compris entre les deux sites EcoRI dans le vecteur pHV1432 (S.D. EHRLICH et al, INRA, Jouy-en-Josas, FRANCE)) conduit également à un plasmide qui ne complémente pas une souche sacU$^-$. Au contraire des expériences précédentes, le sous-clonage à partir du plasmide pBU14 par digestion avec l'enzyme de restriction SphI a permis d'obtenir un plasmide pBU16 contenant un fragment SalI-SphI de 2,55 kb issu de B.subtilis BD1238, capable de restaurer la synthèse de la lévane-saccharase chez un mutant sacU$^-$. Donc le fragment SalI-SphI de 2,55 kb, porté par le plasmide pBU16, comprend le locus sacU et plus particulièrement la région fonctionnelle du locus sacU de B. subtilis. La séquence nucléotidique de ce fragment correspond à celle présentée à la figure 4

En outre, l'introduction de ce fragment de 2,55 kb par l'intermédiaire d'un vecteur approprié, dans une souche de B. subtilis de phénotype Lvs$^+$, conduit dans cette souche à une surproduction des enzymes exocellulaires qu'elle est normalement apte à synthétiser. En d'autres termes, cette séquence à la capacité de conférer le phénotype Deg$^h$ à ladite souche de B. subtilis.

La méthode de clonage du gène sacU décrite ci-dessus est également applicable au clonage du gène sacU$^h$ en utilisant une souche de B. subtilis comportant une mutation sacU$^h$, notamment à l'aide de la souche de B. subtilis QB136 décrite ci-après.

L'invention concerne par conséquent toute séquence de nucléotides contenue dans un fragment SalI-SphI de 2,55kb, issu du chromosome d'une souche de B.subtilis, ladite séquence étant capable, lorsqu'elle est introduite dans une souche de B. subtilis- ou dans tout autre micro-organisme capable d'exploiter à son profit l'information portée par ce fragment- d'y induire une surproduction d'enzymes endo- ou exocellulaires ou de toute autre protéine.

D'une façon générale, l'invention concerne toute séquence homologue du fragment SalI-SphI de 2,55 kb sus-mentionné, cette séquence étant elle-même fonctionnelle pour conférer un phénotype Deg$^h$ à une souche de B.subtilis, cette séquence étant en particulier apte à s'hybrider au fragment de 2,55 kb défini ci-dessus, et ce lorsque les opérations d'hybridation comporte les étapes suivantes :
- pré-traitement (pré-hybridation) du filtre de nitrocellulose supportant le fragment d'acide nucléique à tester avec le tampon d'hybridation (5 x SSC, 1 x Denhardt, 0,1 % SDS), cette opération étant effectuée à 42°C pendant 1 heure ;
- remplacement du tampon d'hybridation au contact du support, sur lequel le fragment d'acide nucléique est alors fixé, par du tampon d'hybridation de même composition, et addition de la séquence de 2,55 kb en tant que sonde, notamment marquée radioactivement, et préalablement dénaturée par un traitement à 100°C pendant 5 minutes ;
- incubation dudit fragment d'acide nucléique fixé sur le support dans ce tampon d'incubation avec le fragment de 2,55 kb à 42°C pendant une durée de 16 à 24 heures,
- l'élimination du tampon contenant la sonde non fixée, par lavages successifs avec 1 x 50 ml de 5 x SSC, 0,1 % SDS à 42°C pendant 20 minutes et 2 x 50 ml de 2 x SSC, 0,1 % SDS pendant 2 x 20 minutes à 42°C.

Il est à rappeler que la composition de la solution de Denhardt est la suivante : 1 % Ficoll, 1 % polyvinylpyrrolidone, 1 % BSA (albumine de sérum de boeuf), et que 1 x SSC est constitué de 0,15 M de NaCl et 0,015 M de citrate de sodium, pH 7.

Les conditions qui précèdent correspondent en particulier à des conditions non stringentes.

L'invention concerne plus particulièrement les séquences homologues conférant aux souches de B. subtilis un phénotype Deg$^h$ et qui s'hybrident dans des conditions stringentes avec le fragment de 2,55 kb, ces conditions d'hybridation comprenant les étapes suivantes :
- traitement de pré-hybridation du filtre de nitrocellulose supportant le fragment d'acide nucléique à tester à 42°C pendant 1 heure avec un tampon ayant la composition suivante : 50 % formamide, 5 x SSC, 1 x Denhardt ;
- remplacement du tampon d'hybridation au contact du support, sur lequel le fragment d'acide nucléique est alors fixé, par du tampon d'hybridation de même composition, et addition de la séquence de 2,55 kb en tant que sonde, notamment marquée radioactivement, et préalablement dénaturée par un traitement à 100°C pendant 5 minutes ;
- une incubation à 42°C pendant une durée de 16 à 24 heures, et
- lavages successifs avec les solutions suivantes :
1 x 50 ml, 5 x SSC, 50 % formamide, 20 minutes à 42°C
2 x 50 ml, 2 x SSC, 0,1 % SDS, 2 x 20 minutes à 42°C
1 x 50 ml, 0,2 x SSC, 0,1 % SDS, 20 minutes à 42°C.

Le fait qu'une séquence d'ADN qui après ce traitement reste hybridée à la séquence d'ADN de 2,55 kb plus particulièrement définie en rapport avec la figure 4 indique que les deux séquences sus-mentionnées possèdent un certain degré d'homologie.

En ce qui concerne le protocole à suivre avant de procéder aux expériences d'hybridation du fragment SalI-SphI de 2,55 kb, on peut procéder de toute façon bien connue de l'homme du métier.

A titre d'exemple, on définit ci-après les conditions dans lesquelles cette fixation peut être effectuée :
- le marquage radioactif du fragment par translation de césure (nick-translation),
- transfert de l'ADN à tester sur un filtre de nitrocellulose,
- dépôt de ce filtre sur du papier Whatmann n°3, imbibé de NaOH 0,5 N, NaCl 0,9 M et incubation pendant 20 minutes à température ambiante,
- répétition de l'opération sur papier imbibé de Tris-HCl M, pH 7, 10 minutes,
- séchage bref,
- répétition de l'opération sur papier imbibé de 2 x SSC,
- séchage sur papier, puis à l'étuve à 80°C pendant 2 heures, permettant la fixation de l'ADN à tester sur le filtre.

Il est à remarquer que les opérations d'hybridation qui précèdent sont avantageusement directement effectuées dans des conditions stringentes, s'agissant d'identifier la séquence sacU ou sacU$^h$ dans une souche de B. subtilis. Cette technique est appliquée particulièrement à l'isolement de la séquence sacU$^h$ d'une souche de B. subtilis sacU$^h$.

En revanche, les conditions non stringentes sont en tout cas recommandées dans les procédés opératoires s'agissant d'isoler une séquence fonctionnelle à partir d'un autre micro-organisme, notamment du type Bacillus, par exemple B. licheniformis.

Des souches de B.subtilis présentant des mutations au niveau du gène sacU ont été obtenues par mutagenèse, notamment selon la méthode décrite dans Lepesant et al (1972) précédemment cité.

Le séquençage des mutations est réalisé suivant la technique PCR (Polymerase Chain Reaction) décrite dans Mullis K.B. et al, Methods Enzymol. 155, 335-350 (1987).

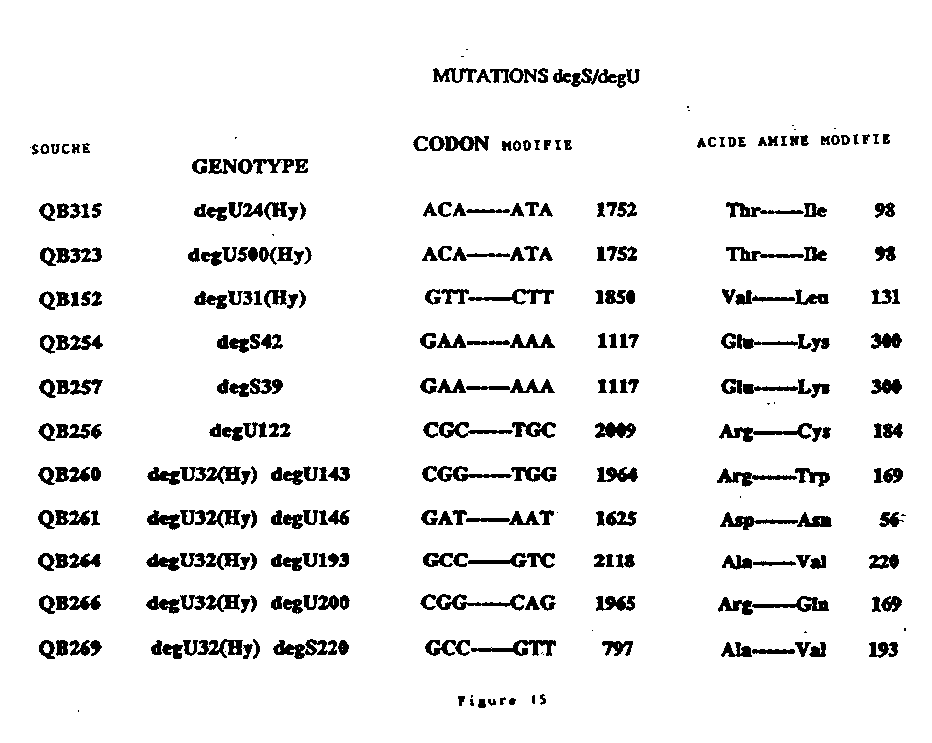

Les mutants ainsi obtenus présentent dans leur génome des mutations représentées sur la figure 15. Parmi ces mutants, deux se sont révélés particulièrement intéressants ; il s'agit de ceux présentant les génotypes degU24 (ou degU500) et degU32 (ou sacU$^h$32) conduisant au phénotype Deg$^h$.

L'invention concerne plus particulièrement encore des acides nucléiques comportant le locus sacU$^h$, en particulier un fragment d'environ 14 kb issu d'une souche de B. subtilis porteuse d'une mutation sacU$^h$.

Ce fragment est capable de s'hybrider, notamment dans les conditions de stringence définies ci-dessus, au fragment d'ADN de 2,55 kb portant l'allèle sacU$^+$ sus-mentionné.

Le clonage de l'allèle sacU$^h$ a été réalisé en introduisant la mutation com-524 de la souche de B. subtilis BD1238 dans la souche de B. subtilis QB136 portant la mutation sacU$^h$32. Un transformant résistant à l'érythromycine (Em$^R$) ayant retenu la mutation sacU$^h$32 a été isolé. Cette souche, appelé QB4209, contient donc à la fois la mutation com-524 et la mutation sacU$^h$32.

Des fragments de restriction BamHI de l'ADN chromosomique de la souche QB4209 ont été insérés dans le site BamHI de pHV1436. Un plasmide recombinant résistant à la fois au chloramphenicol (Cm$^R$) et à l'érythromycine (Em$^R$) a été sélectionné et appelé pBUHI. Ce plasmide est capable de restaurer la synthèse de la lévanesaccharase chez un mutant sacU$^-$ (B. subtilis QB254).

L'invention concerne donc encore un fragment d'acide nucléique, issu d'une souche de B. subtilis portant une mutation sacU$^h$, contenant une séquence fonctionnelle de nucléotides, comportant au plus 14kb, et comprenant la séquence de 2,55 kb sus-mentionnée dans laquelle un au moins des nucléotides est modifié sous l'effet de ladite mutation, ladite séquence fonctionnelle étant caractérisée en ce que :

- elle porte le locus sacU$^h$, codant pour le génotype sacU$^h$,
- elle est capable de restaurer l'activité de synthèse de lévane-saccharase chez une souche de B. subtilis portant une mutation sacU$^-$, lorsque ce fragment a été incorporé à cette souche,
- elle est capable d'induire une surproduction de protéines, ou de conférer un phénotype Deg$^h$, chez B. subtilis- ou chez un autre micro-organisme, capable d'exploiter à son profit l'information portée par ce fragment- lorsque ladite séquence est introduite dans ce micro-organisme,
- elle est capable de s'hybrider-- et de rester hybridée-- avec un fragment d'ADN Sall-SphI de 2,55 kb issu de B. subtilis BD1238, notamment dans les conditions décrites ci-dessus.

Il apparaîtra immédiatement que l'homme de métier peut, par le traitement complémentaire soit des fragments de 2,55 kb et de 14 kb sus-spécifiés, soit d'une séquence homologue à ces fragments, par les enzymes de restriction appropriées, notamment par une digestion de ses extrémités au moyen d'une enzyme exonucléolytique telle que Bal31, produire des fragments plus petits à partir de ces fragments, et tester à nouveau, notamment dans les conditions exposées plus loin, leur capacité à conférer le phénotype Deg$^h$ à une souche de B. subtilis. En d'autres termes, l'homme du métier est désormais à même de localiser avec précision les parties actives des loci sacU et sacU$^h$.

L'étude de la séquence nucléotidique entière du susdit fragment d'ADN de 2,55 kb, issu de B.subtilis ne présentant pas de mutation sacU$^h$, montre que ce fragment d'ADN comprend deux phases ouvertes de lecture.

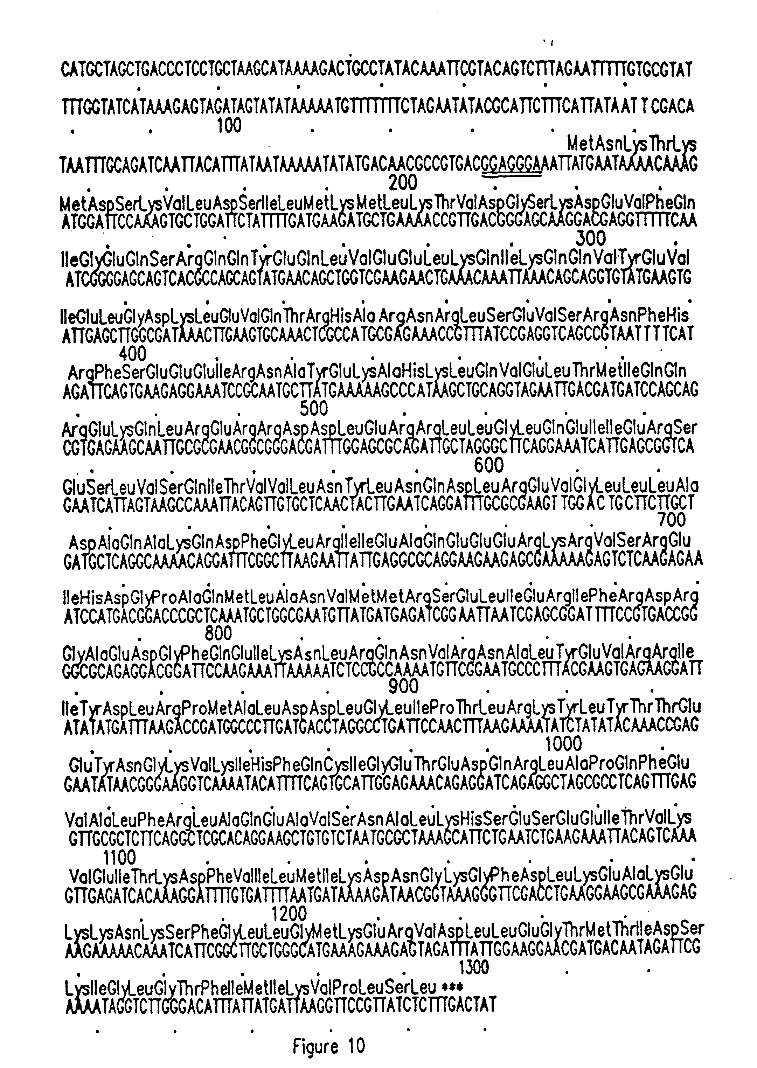

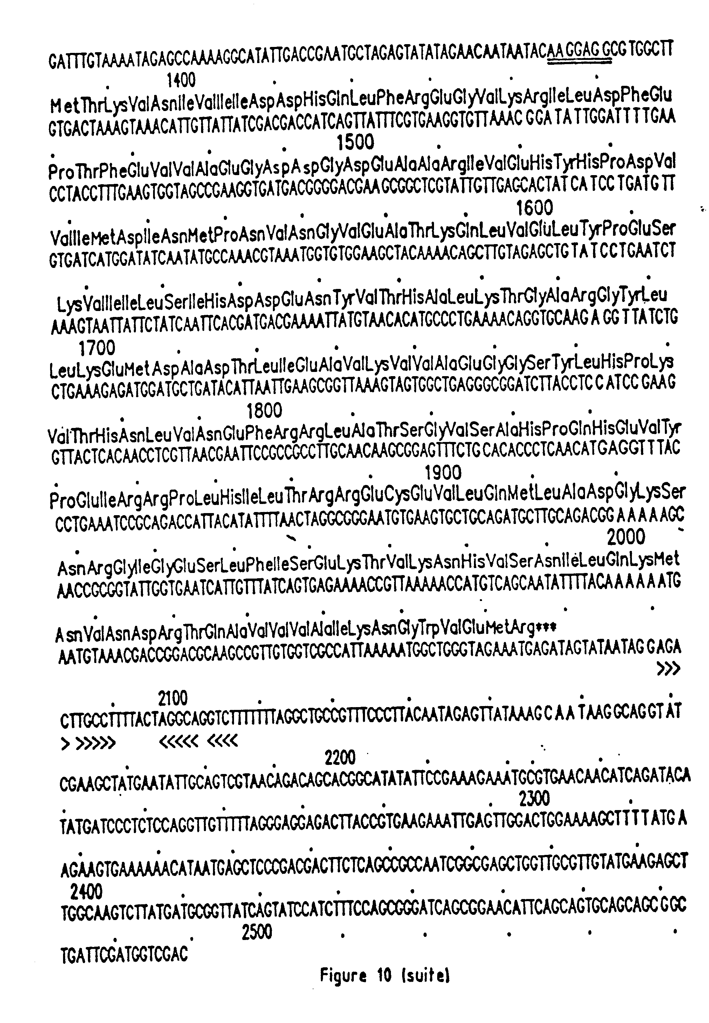

La première phase ouverte de lecture est délimitée par les nucléotides situés aux positions 220 et 1374 de la figure 10, et code pour un polypeptide (ci-après dit SacU$_{S1}$, ou DegS) de 385 acides aminés. Cette phase ouverte de lecture sera encore désignée dans ce qui suit par l'expression ORF1.

La seconde phase ouverte de lecture (ci-après désigné par ORF2) est délimitée par les nucléotides situés aux positions 1460 et 2146 de la figure 10, et code par un polypeptide, (ci-après dit sacU$_{S2}$ ou DegU) de 229 acides aminés (et qui correspond au polypeptide SacU$_S$ décrit dans la demande de brevet France n° 8803736 du 22/03/88 et dont la présente demande revendique la priorité).

Ces deux phases ouvertes de lecture ORF1 et ORF2, sont précédées par des séquences nucléotidiques présumées fournir les signaux typiques de liaison de l'ARN messager aux ribosomes (dites séquences Shine-Dalgarno), ces séquences étant respectivement situées dans les régions délimitées d'une part par les nucléotides situés aux positions 209 et 215, et d'autre part, par les nucléotides situés aux positions 1445 et 1451 de la figure 10.

Aucun signal de terminaison de la transcription n'a pu être déterminé entre ORF1 et ORF2. Par conséquentuent, ORF1 et ORF2 pourraient former, ou faire partie, d'un opéron. ORF2 paraît être suivie d'un terminateur de transcription compris entre les nucléotides situés aux positions 2159 et 2184 de la figure 10.

On doit noter que la seconde phase ouverte de lecture est située de part et d'autre de l'un des sites EcoRI. Or la délétion de la séquence comprise entre ces deux sites supprime l'activité sacU du fragment cloné. On voit que cette délétion élimine plus de la moitié de la séquence codante.

Par conséquent, le polypeptide SacU$_{S2}$, semble nécessaire pour l'activation de la synthèse des enzymes de dégradation chez B. subtilis, puisque l'inactivation de ORF2 (par insertion au niveau du site EcoRI) conduit à un phénotype Lvs$^-$ (déficience en lévane-saccharase).

Il semble que l'ORF1 ne permet pas lorsqu'elle est introduite chez une souche de B. subtilis portant la mutation sacU$^-$ 42 de restaurer l'activité de synthèse d'enzymes de dégradation. Toutefois, l'inactivation de ORF1 (par insertion dans cette dernière d'un ADN étranger) chez une souche de B. subtilis sacU$^+$, conduit également à un phénotype Lvs$^-$.

Il est par conséquent probable que le produit codé par l'ORF1 soit nécessaire, en addition au produit codé par l'ORF2, pour l'obtention du phénotype Deg$^h$ chez B. subtilis.

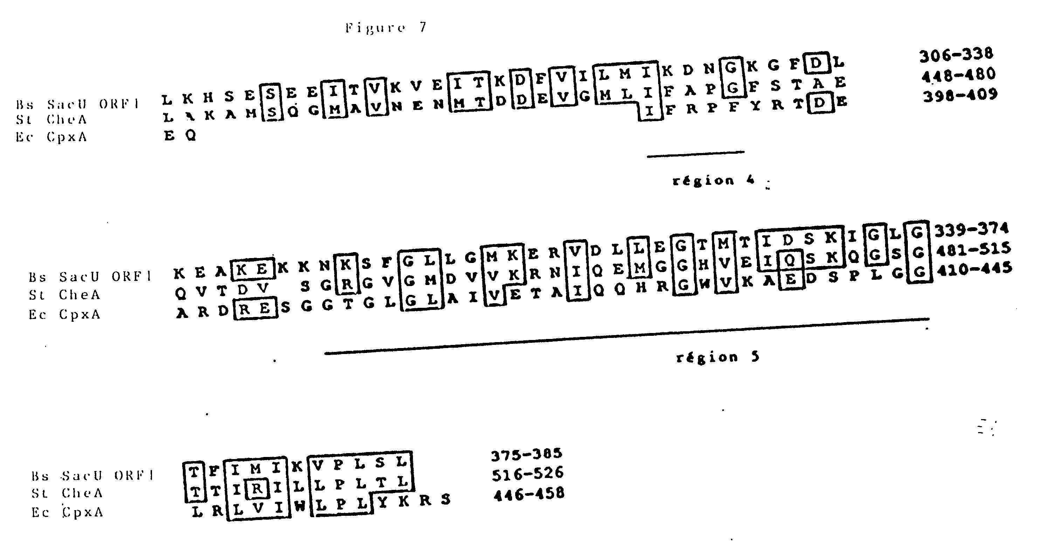

L'analyse de la séquence en acides aminés du polypeptide SacU$_{S1}$ codé par l'ORF1, montre que cette séquence présente une certaine homologie avec celles des protéines qui sont des capteurs de signaux, notamment avec la protéine CheA de S.typhimurium (figure 7), et qui transmettent à des activateurs, notamment ceux sus-mentionnés, l'information nécessaire à l'activation de la transcription.

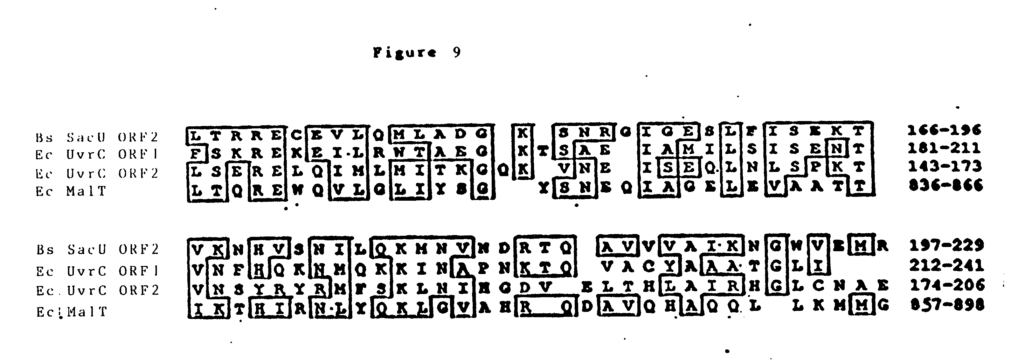

L'analyse de la séquence en acides aminés du polypeptide SacU$_{S2}$ codé par l'ORF2, montre que cette séquence présente une homologie avec celles de plusieurs protéines qui sont des activateurs de transcription, notamment des protéines de E. coli, Dye, OmpR, ou des protéines de B. subtilis, SpoOA, SpoOF (TRACH et al, Proc. Natl. Acad. Sci., 82 : 7260-7264 (1985) (figure 9). On peut en déduire que la protéine SacU$_{S2}$ appartient vraisemblablement à cette famille de protéines, activateurs spécifiques de transcription.

Les polypeptides SacU$_{S1}$ et SacU$_{S2}$ fonctionnent probablement suivant un système dans lequel SacU$_{S1}$ est un capteur de changements spécifiques de l'environnement et qui transmet cette information par l'intermédiaire de SacU$_{S2}$ au niveau du système de transcription.

Pour les besoins de la représentation formelle des homologies de structure partielle entre SacU$_{S1}$, SacU$_{S2}$ et les autres protéines apparaissant au niveau des figures 7 à 9, certains acides aminés se trouvent quelquefois davantage écartés de ceux qui normalement les jouxtent, de façon à permettre la mise en évidence (grâce à des encadrements verticaux) desdites homologies partielles.

L'étude de la séquence nucléotidique des fragments d'ADN de 2,55 kb, issus des différentes souches de B.subtilis sacU$^h$ montre que ces fragments comprennent également deux phases ouvertes de lecture codant pour des polypeptides sensiblement identiques à SacU$_{S1}$ et SacU$_{S2}$, mais qui différent de ces derniers d'au moins un acide aminé.

D'une manière générale, la mutation intervient soit au niveau de la séquence nucléotidique codant pour SacU$_{S1}$, soit au niveau de celle codant pour SacU$_{S2}$.

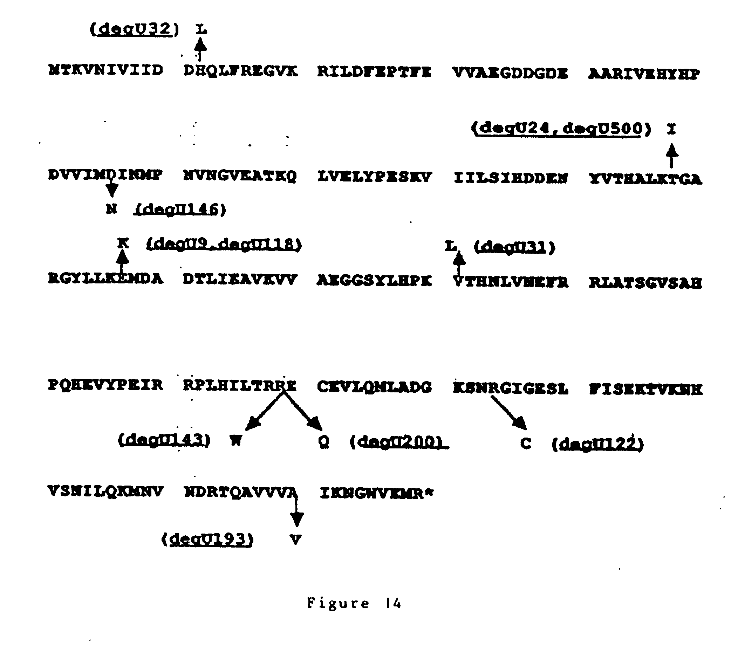

Parmi ces polypeptides, on distinguera notamment le polypeptide DegU24,ou encore DegU32 codé par le fragment de 14 kb sus-mentionné (représentés sur la figure 14) qui conduisent au phénotype Deg$^h$ (notamment en association avec DegS).

L'invention concerne toute séquence d'ADN issue du fragment SalI-SphI de 2,55 kb provenant d'une souche de B.subtilis présentant, ou non, une mutation sacU$^h$, ou encore du fragment de 14 kb mentionné plus haut, et plus particulièrement les séquences codant pour les polypeptides SacU$_{S1}$, SacU$_{S2}$ ainsi que pour tout polypeptide codé par sacU$^h$ (ou encore le polypeptide SacU$_{S1}$$^h$), SacU$^h$$_{S2}$, encore désignés par l'expression "SacU$_s$$^h$") capables de conférer le phénotype Deg$^h$, ainsi que les séquences nucléotidiques comprenant les éléments de régulation de l'expression de ces derniers.

L'invention a également pour objet tout acide nucléique recombinant contenant la partie fonctionnelle de sacU ou celle de sacU$^h$, contenues respectivement dans le fragment SalI-SphI de 2,55 kb issu d'une souche de B;subtilis ne présentant pas de mutation sacU$^h$, ou présentant, au contraire, cette mutation sacU$^h$ (notamment issu du fragment de 14 kb sus-défini, en particulier le fragment du type sus-indiqué codant pour un polypeptide conduisant au phénotype Deg$^h$, notamment pour les polypeptides SacU$_{S1}$ et SacU$_{S2}$, ou encore SacU$_s$$^h$ , ces fragments de l'invention étant associés avec des fragments d'acide nucléique hétérologues vis-à-vis desdits fragments, c'est-à-dire des fragments d'acide nucléique qui peuvent avoir un effet en combinaison avec la séquence codant pour les polypeptides, par exemple des séquences d'acide nucléique susceptibles de contenir un promoteur fort ou une séquence régulatrice ayant pour effet d'augmenter la production des polypeptides susmentionnés, sauvages ou modifiés, dans l'hôte utilisé.

Dans ce qui suit, et pour la commodité du langage, il est entendu que l'expression "fragment d'ADN selon l'invention" recouvre aussi bien les fragments ou les séquences qui ont effectivement une taille de 2,55 kb ou de 14 kb, que les sous-fragments ou sous-segments contenant respectivement, soit la séquence codant pour SacU$_{S1}$,SacU$_{S2}$, ou SacU$_s$$^h$, soit la séquence codant pour une partie de ces polypeptides néanmoins fonctionnelle dans les conditions qui ont été indiquées.

L'invention a également pour objet un fragment d'ADN tel que défini ci-dessus, caractérisé par des substitutions de certains de ses nucléotides par d'autres sans que soit modifiée pour autant l'activité du, ou des, polypeptide(s) qui lui corresponde(nt).

L'invention concerne tout ADN recombinant contenant un fragment d'ADN selon l'invention, conférant le phénotype Deg$^h$, associé avec un promoteur de transcription reconnu par une cellule hôte, autre que celle dont ledit fragment d'ADN est issu. Avantageusement, le susdit promoteur utilisé est un promoteur fort tel que le promoteur du gène sacB (A. KLIER et al, Molec. Microbiol., 1 : 233-241 (1987)), ou le promoteur Pn25 du bactériophage T$_5$ (M. ZUKOWSKI et L. MILLER, Gene, 46 : 247-255 (1986)). Avantageusement, cet ADN recombinant comprend un fragment d'ADN selon l'invention, un promoteur, notamment un promoteur fort, et, le cas échéant, un terminateur de transcription hétérologue par rapport à l'ADN de B. subtilis, ce promoteur et, le cas échéant, ce terminateur étant néanmoins reconnus par la cellule hôte, dans des conditions permettant au susdit fragment d'ADN selon l'invention d'y être exprimé, lorsque ledit ADN recombinant y aura été introduit. Par exemple, cet ADN recombinant comprend un promoteur et, le cas échéant, un terminateur d'un autre micro-organisme.

A cet égard, l'invention concerne plus particulièrement les ADN recombinants consistant en des vecteurs, contenant un fragment d'ADN selon l'invention en l'un de leurs sites non essentiels pour leur réplication, notamment du type plasmide, qui sont aptes à se répliquer dans une cellule hôte tout en permettant à la partie fonctionnelle du locus sacU, ou du locus sacU$^h$, de manifester son activité, notamment à permettre l'expression des séquences codant pour les polypeptides SacU$_{S1}$, SacU$_{S2}$ ou SacU$_s$$^h$.

Avantageusement, le susdit promoteur de transcription est un promoteur inductible. A titre d'exemple de promoteur inductible, on citera le promoteur de sacB inductible par le saccharose (SHIMOTSU et HENNER, J. Bacteriol., 168, 380-388 (1986), KLIER et al., Molec. Microbiol. (1987) précédemment cité), et le promoteur spac (Proc. Natl. Acad. Sci. USA, 81, 439-443, (1984)) inductible par l'IPTG (isopropyl β-D-thiogalactoside).

Les vecteurs selon l'invention peuvent être choisis parmi ceux capables de se répliquer dans la cellule hôte, c'est-à-dire sans pour autant modifier le chromosome de ladite cellule (tels que les plasmides multicopies capables de se multiplier en un grand nombre de copies à l'intérieur de la cellule hôte). La transformation d'une cellule hôte par les vecteurs du type réplicatif sus-mentionnés conduit à l'introduction d'une information génétique supplémentaire dans le génome de la cellule, mais pas dans son chromosome.

Des vecteurs intégratifs capables de modifier le chromosome de la cellule hôte peuvent également être utilisés. De tels vecteurs permettent l'insertion d'un fragment d'ADN selon l'invention dans le chromosome de la cellule hôte, notamment par un mécanisme de simple ou de double récombinaison.

D'une manière générale, l'ensemble des vecteurs faisant l'objet de la description qui suit sont soit du type réplicatif, soit du type intégratif.

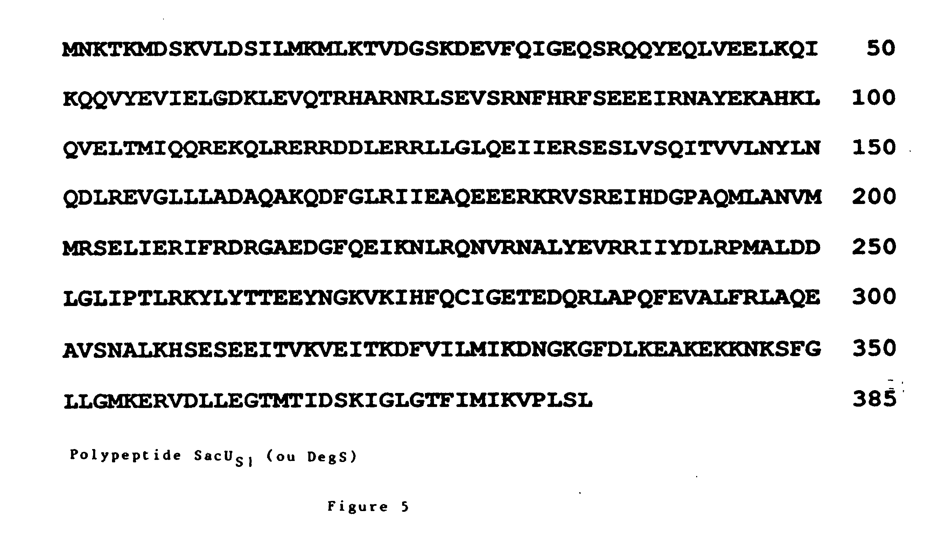

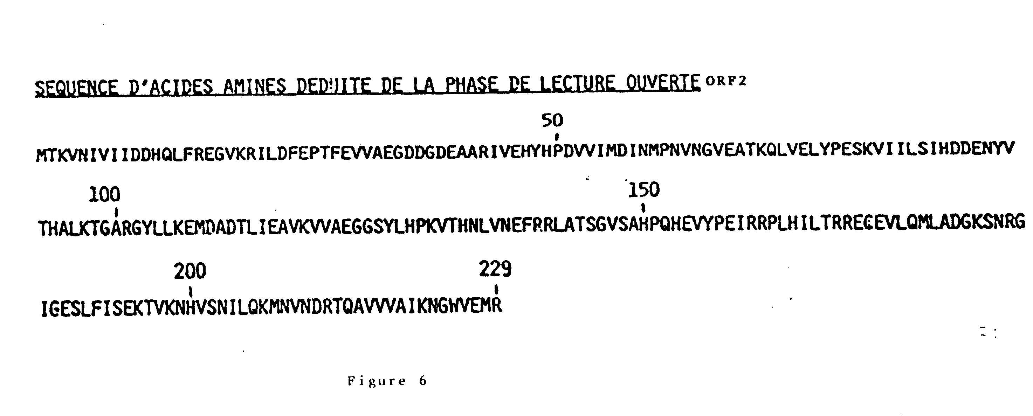

L'invention concerne également les polypeptides SacU$_{S1}$$^h$ et SacU$_{S2}$$^h$ représentés sur les figures 5 et 6 ainsi que les fragments peptidiques actifs qui en découlent, en tant qu'ils sont capables d'activer l'expression d'un, ou de plusieurs gènes de structure de l'organisme, notamment de type Bacillacées, qui les héberge.

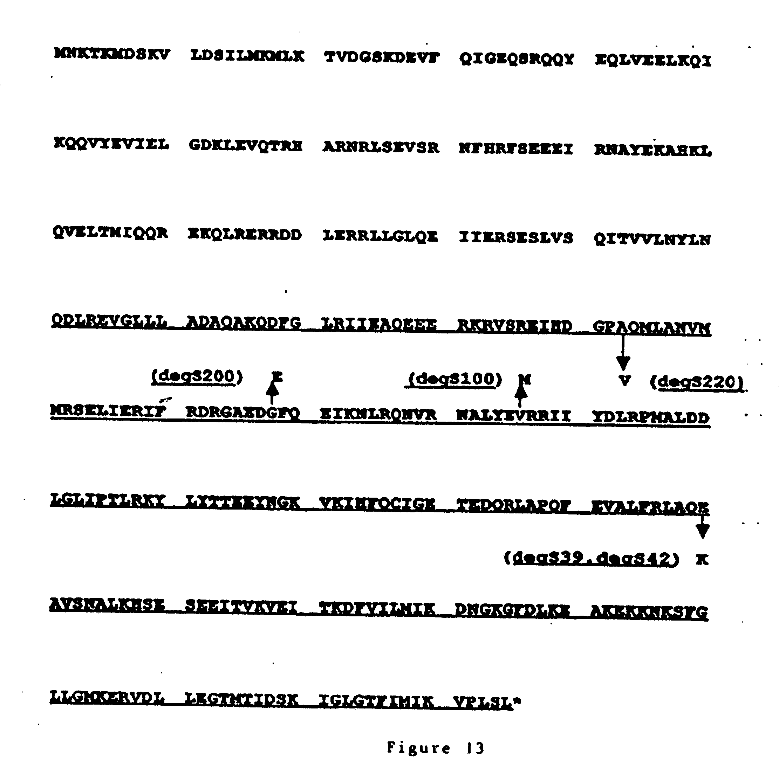

L'invention concerne également les polypeptides SacU$^h$$_{S1}$ et SacU$^h$$_{S2}$ représentés respectivement sur les figures 13 et 14 et les fragments peptidiques actifs qui en découlent, en tant qu'ils sont capables d'activer l'expression d'un, ou de plusieurs gènes de structure de l'organisme, notamment de type Bacillacées qui les héberge.

L'invention a également pour objet un polypeptide hybride obtenu par fusion de toute ou partie d'un fragment d'ADN selon l'invention, notamment par fusion de toute ou partie de la séquence codant pour SacU$_{S1}$ ou pour SacU$_{S2}$, ou pour SacU$_S$$^h$ et d'un fragment d'ADN hétérologue de manière à produire dans la cellule hôte un polypeptide hybride composé, d'une part, de toute ou partie du polypeptide codé par le fragment d'ADN selon l'invention, notamment SacU$_{S1}$, SacU$_{S2}$, ou SacU$_S$$^h$ et, d'autre part, une séquence d'acides aminés hétérologue.

L'invention concerne donc un procédé visant à accroître la capacité de production de protéines, notamment d'enzymes ou de polypeptides, par un micro-organisme approprié, ce procédé comprenant la transformation des cellules dudit micro-organisme au moyen d'un des plasmides sus-indiqués, contenant un fragment d'ADN selon l'invention, la mise en culture des cellules hôtes transformées dans un milieu de culture approprié et la récupération desdites protéines, soit à partir de ces cellules (notamment par lyse des cellules dans le cas de la production de protéines endocellulaires), soit à partir du milieu de culture (dans le cas de la production d'enzymes exocellulaires).

Parmi les micro-organismes transformables par le procédé sus-mentionné, ainsi que par les autres procédés selon l'invention décrits ci-après, on distinguera notamment les bactéries Gram$^+$, telles que les genres Bacillus et Clostridium.

Les protéines ainsi produites sont celles dont les gènes de structure sont naturellement sous le contrôle du gène sacU, ou du gène sacU$^h$, dans le chromosome des cellules bactériennes utilisées pour la mise en oeuvre du procédé selon l'invention.

Par exemple, un tel procédé selon l'invention permet la surproduction, par B. subtilis, d'un grand nombre d'enzymes endo- ou exocellulaires, dont par exemple les protéases, la lévane-saccharase, l'$\alpha$-amylase et des glycanases.

L'invention vise également un procédé de production d'un polypeptide déterminé par un micro-organisme approprié dont le génome, ou le chromosome, a été modifié de manière à ce que le génome, ou le chromosome, contienne une séquence nucléotidique codant pour ledit polypeptide dont la production doit être stimulée, qui comprend la transformation des cellules dudit micro-organisme au moyen d'un des plasmides sus-indiqués, contenant un fragment d'ADN selon l'invention, la mise en culture des cellules du micro-organisme ainsi transformées dans un milieu approprié, et la récupération dudit polypeptide à partir desdites cellules ou du milieu de culture.

L'effet de contrôle par le locus sacU, ou le locus sacU$^h$, de la synthèse des enzymes chez les micro-organismes utilisés pour la mise en oeuvre des procédés de l'invention sus-mentionnés, est probablement le résultat de l'action sur une séquence cible, comprise dans le génome desdits micro-organismes, soit d'un ou des polypeptides codés par un fragment d'ADN selon l'invention, notamment des polypeptides SacU$_{S1}$, SacU$_{S2}$ ou SacU$_{S1}$$^h$ et SacU$_{S2}$$^h$, soit d'un autre produit exprimé sous le contrôle des dits polypeptides.

Les séquences cibles sus-mentionnées sont généralement situées en amont des gènes de structure codant pour les enzymes secrétées par B. subtilis.

A titre de séquence cible, on peut citer notamment :
- celle comprise dans un fragment de 440 paires de bases, délimité par les sites de restriction Sau3A1-Rsa1, et situé en amont du site de liaison avec les ribosomes et de la séquence d'ADN codant pour la lévane-saccharase chez B. subtilis,
- celle comprise dans une région d'environ 200 paires de bases située en amont du début de la transcription du gène de la protéase alcaline (HENNER et al, J.Bacteriol., 170 : 296-300 (1988)).

La cellule hôte utilisée par la mise en oeuvre du procédé sus-mentionné est donc transformée, d'une part, au moyen d'un vecteur, notamment plasmide, contenant, en l'un de ses sites non essentiels pour sa réplication, une séquence nucléotidique codant pour le polypeptide déterminé, précédée par une séquence nucléotidique comprenant une séquence cible et les éléments nécessaires pour permettre l'expression de la séquence codant pour le polypeptide déterminé, et, d'autre part, au moyen d'un des vecteurs sus-indiqués contenant un fragment d'ADN selon l'invention.

Selon un autre mode de réalisation d'un tel procédé de production d'un polypeptide déterminé selon l'invention, la cellule hôte utilisée dans ledit procédé est transformée à l'aide d'un plasmide contenant à la fois, en l'un de ses sites non essentiels pour sa réplication, un fragment d'ADN selon l'invention et une séquence nucléotidique codant pour le polypeptide déterminé, précédée par une séquence nucléotidique comprenant une séquence cible et les éléments nécessaires pour permettre l'expression de la séquence codant pour la synthèse du polypeptide déterminé, plus particulièrement à l'intérieur de la cellule.

D'une manière générale, toute protéine naturellement secrétée par une bactérie Gram+, notamment du genre Bacillus, est tout d'abord traduite sous forme d'un polypeptide précurseur dont la séquence en acides aminés correspond à celle d'un peptide signal suivie de celle de la protéine secrétée. Au moment de la secrétion cellulaire, seule la forme mature correspondant à la protéine elle-même est secrétée hors de la cellule.

Ainsi l'invention concerne également un procédé de production d'un polypeptide déterminé qui comprend la transformation de la cellule hôte de manière à ce que le génome, ou le chromosome, de cette dernière contienne une séquence codant pour un polypeptide déterminé, précédée par une séquence signal, ainsi qu'un fragment d'ADN selon l'invention, la mise en culture desdites cellules dans un milieu approprié et la récupération du polypeptide déterminé à partir du milieu de culture dans lequel il est secrété par le micro-organisme utilisé dans ledit procédé.

La cellule hôte utilisée pour la mise en oeuvre d'un tel procédé est transformée, d'une part, au moyen d'un vecteur, notamment plasmide, contenant, en l'un de ses sites non essentiels pour sa réplication, la séquence d'ADN codant pour le polypeptide déterminé, précédée d'une séquence signal, qui est elle-même précédée par une séquence cible et par une séquence nucléotidique comprenant les éléments nécessaires pour permettre l'expression de la séquence signal et de la séquence codant pour le polypeptide déterminé, et d'autre part par un des plasmides sus-indiqués contenant un fragment d'ADN selon l'invention.

L'invention a également pour objet un procédé de production d'un polypeptide déterminé par un micro-organisme approprié qui comprend la transformation des cellules dudit micro-organisme au moyen d'un vecteur contenant à la fois, en l'un de ses sites non essentiels pour sa réplication, un fragment d'ADN selon l'invention, codant pour un activateur conduisant au phénotype Deg$^h$, et une séquence d'ADN codant pour le polypeptide déterminé, cette dernière séquence d'ADN étant précédée par une séquence signal, qui est elle-même précédée par une séquence cible et par une séquence nucléotidique comprenant les éléments nécessaires pour permettre l'expression de la séquence signal et de la séquence codant pour le polypeptide déterminé, la mise en culture desdites cellules transformées dans un milieu approprié, et la récupération dudit polypeptide à partir du milieu de culture.

Avantageusement les cellules hôtes utilisées pour la mise en oeuvre de l'ensemble des procédés de production de protéines sus-mentionnés, sont également transformées à l'aide d'un vecteur, notamment plasmide, contenant, en l'un de ses sites non essentiels pour sa réplication, une ou plusieurs séquences d'ADN codant pour un autre activateur conduisant au phénotype Deg$^h$ que celui codé par un fragment d'ADN selon l'invention, et les éléments nécessaires pour permettre l'expression de ladite séquence d'ADN.

Parmi les activateurs du phénotype Deg$^h$ indiqués ci-dessus, on citera notamment les polypeptides SacQ$_L$, SacQ$_S$, SacQ$_A$, codés respectivement par les gènes sacQ de B. licheniformis, B. subtilis et B. amyloliquefaciens, et le polypeptide de 60 acides aminés codé par le gène prtR de B. natto.

Avantageusement, le vecteur sus-mentionné comprend une séquence d'ADN codant pour le polypeptide SacQ$_L$.

Les transformations de la cellule hôte de manière à ce que son génome, ou son chromosome, contienne une, ou plusieurs, séquence(s) d'ADN codant pour un activateur autre que celui codé par un fragment d'ADN selon l'invention, peut également être réalisée par l'intermédiaire de tout vecteur utilisé pour la mise en oeuvre de l'ensemble des procédés de l'invention décrits ci-dessus, ledit vecteur contenant un autre fragment d'ADN codant pour un, ou plusieurs, des activateurs sus-mentionnés.

A ce titre, l'invention concerne plus particulièrement un vecteur, notamment plasmide, contenant, en l'un de ses sites non essentiels pour sa réplication, un fragment d'ADN selon l'invention, ainsi qu'un fragment d'ADN codant pour un, ou plusieurs, activateur(s) conduisant au phénotype Deg$^h$ autre(s) que celui codé pour le fragment d'ADN selon l'invention, et contenant les éléments nécessaires pour permettre l'expression de ce dernier fragment d'ADN.

L'invention concerne aussi le vecteur sus-mentionné contenant également un fragment d'ADN contenant une séquence d'ADN codant pour un polypeptide déterminé, précédée par une séquence d'ADN contenant une séquence cible et les éléments nécessaires pour permettre l'expression de la séquence codant pour ledit polypeptide.

L'invention concerne plus particulièrement le vecteur tel que décrit ci-dessus et comprenant également en amont de la séquence codant pour le polypeptide déterminé, une séquence signal ainsi que les éléments nécessaires pour permettre l'expression de la séquence signal et de celle codant pour ledit polypeptide.

Avantageusement, le vecteur sus-mentionné comprend une séquence d'ADN codant pour le polypeptide activateur SacQ$_L$.

Parmi les séquences signal utilisées, pour la construction des vecteurs de l'invention, on citera notamment celle permettant la secrétion de la protéase alcaline, de la lévane-saccharase, de la cellulase et de l'α-amylase.

D'une manière générale, toute séquence pouvant jouer le rôle de séquence signal précédant la séquence d'ADN codant pour une enzyme dans un micro-organisme approprié peut être utilisée.

L'invention concerne également les cellules de B. subtilis, ou de tout autre micro-organisme approprié, transformées par les plasmides sus-mentionnés comprenant un fragment d'ADN selon l'invention conduisant au phénotype Deg$^h$, notamment un fragment d'ADN codant pour le polypeptide SacU$_{S1}$, et/ou SacU$_{S2}$ ou encore SacU$_S{}^h$, ces plasmides étant capables de se répliquer dans ces cellules.

L'invention concerne plus particulièrement les cellules bactériennes sus-mentionnées qui sont transformées par un plasmide contenant un fragment d'ADN selon l'invention et une, ou plusieurs, séquence(s) codant pour un autre activateur conduisant au phénotype Deg$^h$ que celui codé par le fragment d'ADN de l'invention.

L'invention a également pour objet les cellules bactériennes transformées par un plasmide tel que décrit ci-dessus et contenant également une séquence codant pour un polypeptide déterminé, éventuellement précédée par une séquence signal, cette dernière étant précédée par une séquence cible, tel que décrit plus haut.

Ces cultures de cellules sont alors capables de produire des quantités accrues de protéines, notamment d'enzymes, et/ou du polypeptide déterminé.

Bien entendu, l'homme du métier appréciera que les polypeptides recherchés, plus particulièrement le polypeptide déterminé, peuvent être purifiés de toute façon en soi connue, par exemple par électrophorèse sur gel et récupération du polypeptide recherché. On peut également encore avoir recours à toute autre technique par exemple à la chromatographie liquide sous haute pression.

L'invention concerne également le fragment d'ADN codant pour le polypeptide SacU$_{S1}$, ainsi que celui codant pour le polypeptide sacU$_{S2}$, lesdits fragments étant compris dans le fragment d'ADN de 2,55 kb sus-menté. L'invention a plus particulièrement pour objet des vecteurs du type de ceux décrits ci-dessus et contenant le fragment d'ADN codant pour SacU$_{S1}$, ou celui codant pour SacU$_{S2}$, en lieu et place du fragment D'ADN de 2,55 kb sus-mentionné, et l'utilisation de tels vecteurs dans des procédés de production de protéines tels que décrits ci-dessus, les génomes des cellules hôtes utilisées dans de tels procédés contenant alors des éléments nécessaires pour permettre au polypeptide SacU$_{S1}$ ou SacU$_{S2}$ d'induire le phénotype Deg$^h$ chez ces cellules. Notamment, lorsque le vecteur sus-mentionné comprend un fragment d'ADN codant pour SacU$_{S1}$, le génome des cellules hôtes utilisées dans ledit procédé contiendra avantageusement un fragment d'ADN codant pour un polypeptide activateur (tel que SacU$_{S2}$) capable de recueillir l'information portée par le polypeptide SacU$_{S1}$ ; et lorsque ledit vecteur comprend un fragment d'ADN codant pour SacU$_{S2}$, le génome desdites cellules contiendra avantageusement un fragment d'ADN codant pour un polypeptide capteur (tel que SacU$_{S1}$) capable de transmettre l'information au polypeptide SacU$_{S2}$ pour l'activation de la transcription.

Ce dernier mode de réalisation du procédé de production de protéines selon l'invention est également réalisable à l'aide de vecteurs comprenant respectivement un fragment d'ADN codant pour un polypeptide de type SacU$_{S1}$$^h$ (ou DegS$^h$) ou SacU$_{S2}$$^h$ (ou DegU$^h$).

L'invention concerne enfin tout polypeptide ayant les mêmes caractéristiques fonctionnelles que celles que manifeste celui produit par le locus sacU$^h$, lorsqu'un vecteur le contenant est introduit dans une cellule ayant compétence pour l'exploiter. Il s'agit de tout polypeptide correspondant à un cadre de lecture ouvert (open reading frame) contenue dans la région sacU$^h$ elle-même.

Des caractéristiques supplémentaires de l'invention apparaîtront encore au cours de la description qui suit des vecteurs préférés et des conditions dans lesquelles ils peuvent être utilisés, étant entendu que cette description ne saurait être interprétée comme tendant à restreindre la portée des revendications.

## 1) Materiels et méthodes

### - Souches bactériennes et plasmides

Les souches de B. subtilis suivantes ont été utilisées : la souche de référence de B. subtilis 168 (trp C2) (sacU$^+$, phénotype Lvs$^+$ Prt$^+$) ; le mutant sacU$^-$ QB254 (sacU42,sacA321,his A1,trpC2) ne synthétisant pas de lévane-saccharase et de protéase (phénotype Lvs-, Prt-) (LEPESANT et al, Mol. Gen. Genet. 118 : 135-160 (1972)) ; le mutant sacU$^h$ QB136 (sacU32,leuA8,trpC2) (KUNST et al, 1974, précédemment cité) la souche 1A510, qui est déficiente en recombinaison intermoléculaire (recE4,leulA8,arg-15,thrA5,stp) (OSTROFF ET PENE, J. Bacteriol., 156 : 934-936 (1983)) ; la souche BD 1238 (com-524:Tn917lacZ hisA1 leu metB5) (HAHN et al, J. Bacteriol., 169 : 3104-3109 (1987) ; ALBANO et al, J. Bacteriol., 169 : 3110-3117, (1987)).

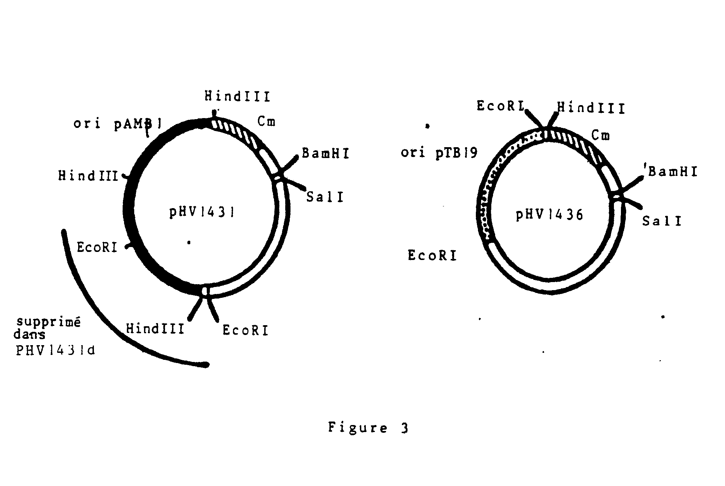

Les vecteurs de clonage pHV1431 et pHV1436 construits par JANNIERE L. et EHRLICH S.D. (INRA, Jouy-en-Josas, FRANCE) et présentés sur la figure 3, permettant de maintenir de manière stable de larges fragments d'ADN chez B. subtilis. Le plasmide pHV1431 (10,8kb ; portant un gène de résistance au chloramphenicol Cm$^R$) contient une origine de réplication provenant du plasmide pAMβ1 (LEBLANC et LEE, J. Bacteriol., 157 : 445-453 (1984)) et est maintenu, à un grand nombre de copies, chez B. subtilis.

Le plasmide pHV1431d est un dérivé de 8,3 kb de pHV1431 obtenu par délétion spontanée chez E. coli, éliminant un fragment d'ADN contenant les deux sites EcoRI de pHV1431.

Le plasmide pHV1436 (8,7 kb, Cm$^R$) contient une origine de réplication provenant du plasmide pTB19 (IMANAKA et al, J. Bacteriol., 146 : 1091-1097 (1981)) et est maintenu à un faible nombre de copies chez B. subtilis.

### - Milieux et tests qualificatifs

E. coli est cultivé dans un milieu L (tryptone 10 g/l, extrait de levure 5 g/l, NaCl 5 g/l) et B. subtilis dans le milieu AM$_3$ (Penassay, DIFCO) ou dans le milieu MMCH qui est composé de K$_2$HPO$_4$ 60 mM, KH$_2$PO$_4$ 44 mM, (NH$_4$)$_2$SO$_4$ 15 mM, citrate de Na$_3$ 3 mM, Mg SO$_4$ 2 mM, MnCl$_2$ 0,01 mM, citrate d'ammonium ferrique 22 mg/l, hydrolysat de caséine 0,05 %, et de facteurs d'auxotrophie (100 mg/l).

Les boites de Petri L ou SP ont été préparées par addition de 17 g/l de Bacto-agar (DIFCO) au bouillon L ou au milieu SP (AMORY et al, J. Bacteriol., 169 : 324-333 (1987), LEPESANT et al (1972) précédemment cité).

Les milieux sélectifs contiennent des antibiotiques aux concentrations suivantes : ampicilline (Ap) 25 µg/ml : chloramphenicol (Cm) 5 µg/ml : erythromycine (Em) 1 µg/ml et 25 µg/ml de lincomycine.

Les recombinants capables de produire de la lévane-saccharase (phénotype Lvs[+]) ont été identifiés sur des boites ST qui contiennent 20 g de saccharose stérilisé par filtration (BDH), 1 g de tryptone (DIFCO), 17 g/l d'agar purifié (DIFCO), 13 mM de KCl et 1 mM de MgSO$_4$. Les milieux STCm et STEm contiennent, respectivement, 5 µg/ml de chloramphenicol et 1 µg/ml d'erythromycine additionné de 25 µg/l de lincomycine. L'aspect muqueux des lévanes entourant les clones Lvs[+] sur les boites ST permet de distinguer ces clones Lvs[+] des clones Lvs[-]

- Détermination de l'activité lévane-saccharase

Des dilutions appropriées de surnageants de cultures sur milieu MMCH sont incubés à 37°C en présence de 10% de saccharose, et 50 mM de tampon phosphate de potassium (pH 6.0) dans un volume final de 1 ml. Les réactions sont arrêtées par ébullition. La quantité de glucose est mesuré à l'aide du réactif GOD-Perid (BOEHRINGER). Une unité correspond à 1 µmole de glucose produit par minute. La quantité totale de protéine est estimée à partir de la densité optique de la culture.

- Transformation des cellules compétentes

Ces transformations sont réalisées à l'aide des méthodes décrites pour la transformation d'E. coli (COHEN et al, Proc. Natl. Acad. Sci., USA, 69 : 2110-2114 (1972)), ou de B. subtilis (ANAGNOSTOPOULOS et SPIZIZEN, J. Bacteriol., 81 : 741-746 (1961) ; NIAUDET et EHRLICH, Plasmid, 2 : 48-58 (1979)).

L'ADN donneur est incubé avec des cellules compétentes de B. subtilis pendant 20 minutes à 37°C.

De l'extrait de levure et des facteurs d'auxotrophie sont alors additionnés à des concentrations finales de 10 mg/ml et 50 µg/ml, respectivement. Après incubation avec agitation à 37°C en présence d'antibiotiques (0,5 µg/ml de chloramphenicol pendant 45 minutes, et 0,5 µg/ml de chloramphenicol plus 0,15 µg/l d'érythromycine pendant 90 minutes), les cellules sont étalées sur des boites SP additionnées soit de chloramphamphenicol seul, soit de chloramphenicol, d'érythromycine et de lincomycine.

- Manipulations d'ADN et procédures de clonage.

L'ADN plasmidique est extrait d'E. coli ou de B. subtilis par la méthode de la lyse alcaline (MANIATIS et al, Molecular Cloning, Cold Spring Harbor Laboratory Press, Cold Spring Harbor New York, (1982) ; AMORY et al (1987) précédemment cité), suivie d'une centrifugation en gradient de chlorure de césium dans le cas d'extractions préparatives à partir de grands volumes de cultures. Les fragments d'ADN sont obtenus à partir de gels d'agarose, notamment par électro-élution.

Les enzymes de restriction et l'ADN-ligase de T4 ont été utilisées suivant les recommandations de leur fabricant respectif.

Les banques de gènes des plasmides recombinants contenant des fragments d'ADN de la souche de B. subtilis BD 1238, ont été construits par ligature in vitro en utilisant des concentrations élevées d'ADN (100 µg/ml), afin de promouvoir la formation de multimères d'ADN qui transforment efficacement les cellules de B. subtilis.

Les plasmides sous forme de monomères ont une faible, voire aucune, activité de transformation (CANOSI et al, Mol. Gen. Genet., 166 : 259-267 (1978)). 2 µg d'ADN de pHV1436 ou pHV1431d linéarisés avec BamHI et 2 µg d'ADN issu de BD1238 digéré par BamHI sont ligaturés en présence d'ADN-ligase de T4 dans des volumes réactionnels de 20 µl. Puisque les fragments d'ADN insérés dans les vecteurs plasmidiques et le chromosome de la souche réceptrice de B. subtilis sont homologues, des recombinaisons entre les deux peuvent avoir lieu dans des souches Rec[+]. Pour cette raison, une souche 1A510 a été utilisée. En effet, la mutation recE4 de cette souche conduit à une déficience en recombinaison intermoléculaire (Rec[-]) mais n'affecte pas la recombinaison intramoléculaire permettant la transformation par des plasmides multimères (entrant dans la cellule) en plasmides monomères (MICHEL et al, EMBO. J., 1 : 1565-1571 (1982) ; TANAKA, J. Bacteriol., 139 : 775-782 (1979). Le plasmide pBU2 est obtenu en utilisant directement le mélange de ligature pour transformer la souche 1A510. pBU3 dérive de pBU2 par transfert de l'insertion BamHI (13,4 kb) de ce dernier au vecteur pHV1431d. Dans un volume réactionnel de 20 µl, l'ADN de pHV1431d linéarisé au préalable par BamHI et l'ADN de pBU2 digéré au préalable par BamHI ont été ligaturés. Le plasmide pBU14 dérive de pBU3 par ligature de 2 µg d'ADN de pBU3 digéré par SalI dans un volume réactionnel de 20 µl. De même, le plasmide pBU16 est construit à partir de pBU14 par ligature de l'ADN de pBU14 digéré par SphI. Ces plasmides sont tous introduits dans deux souches de B. subtilis : la souche QB254 afin de tester leur capacité de restauration de la synthèse de lévane-saccharase dans cette souche sacU[-], et la souche 1A510, pour le maintien des plasmides et leur extraction préparative.

2) Isolement du locus sacU dans le plasmide

Le clonage du locus sacU dans les vecteurs plasmidiques est réalisé à l'aide de la mutation com-524 (HAHN et al, (1987) ; ALBANO et al, (1987), précédemment cités). Cette mutation apparaît par insertion du transposon Tn917lacZ (PERKINS et al, (1986) ; SHAW et al, (1985), précédemment cités) dans une région proche du locus sacU.

Afin de mesurer la liaison génétique existant entre com-524 et sacU, l'ADN de la souche BD1238 a été extrait et utilisé à une concentration finale non saturée pour transformer la souche sacU[-] QB254. 50 % des transformants résistants à l'érythromycine, possédent la capacité de produire la lévane-saccharase (Lvs[+]) sur boites de Pétri STEm et ont donc hérité l'allèle sacU[+], ce qui indique que le locus sacU et le marqueur de résistance à l'érythromycine com-524 sont très proches.

La détermination de cette liaison génétique et le fait que la carte de restriction de Tn917lacZ est connue, ont été très utiles pour le clonage du locus sacU, en particulier, un site BamHI est localisé à proximité de l'extrémité gauche de Tn917lacZ (figure 1).

La procédure de clonage a donc été réalisée en insérant des fragments BamHI de l'ADN chromosomique de BD1238 dans le plasmide pHV1436. Un plasmide recombinant qui confère la résistance à l'érythromycine aux cellules hôtes (Em$^R$) a été sélectionné en utilisant comme souche réceptrice 1A1510. Ainsi, le plasmide recombinant Cm$^R$ Em$^R$ obtenu a été appelé pBU2.

Le plasmide pBU2 contient un fragment BamH1 de l'ADN de BD1238 inséré au niveau du site BamH1 de pHV1436. Ce fragment comprend le transposon Tn917lacZ dans sa presque totalité et des séquences d'ADN situées à la droite de Tn917lacZ.

Afin de déterminer si pBU2 contient l'allèle sacU à proximité du Tn917lacZ, ce plasmide a été utilisé pour transformer le mutant sacU$^-$ QB254. Les cellules résistantes au chloramphenicol ont été sélectionnées. Il a ainsi été déterminé que le plasmide pBU2 contient au moins une partie de l'allèle sacU$^+$, puisque ce plasmide est capable de conférer le phénotype Lvs$^+$ à la souche QB254.

Toutefois, le plasmide pBU2 est difficile à manipuler pour deux raisons. Premièrement, le rendement de ce plasmide est très faible. Après centrifugation en gradient de densité de chlorure de césium, environ 3 µg d'ADN plasmidique est obtenu par litre de culture. Deuxièmement, il n'est pas possible de transférer ce plasmide en entier chez E. coli.

Par conséquent, le fragment BamHI contenant Tn917lacZ et le locus sacU est transféré dans un vecteur qui peut être maintenu à un nombre de copies élevé chez B.subtilis. Le vecteur sus-mentionné correspond au plasmide pHV1431 décrit dans la figure 3. Toutefois, le plasmide pHV1431 est instable chez E. coli, et a par conséquent été remplacé par le plasmide pHV1431d. Ce dernier, est obtenu par élimination d'un fragment chevauchant les deux sites EcoRI de pHV1431, et se maintient de façon stable chez E. coli et chez B. subtilis. L'ADN de pHV1431d est linéarisé au niveau de son site unique BamHI et ligaturé au fragment BamHI contenant le locus sacU de pBU2 pour produire pBU3. Le rendement du plasmide pBU3 est bien meilleur que celui de pBU2 (50 à 100 µg/l de culture).

Le locus sacU de pBU3 a été localisé en sous-clonant un fragment SalI de 3,9 kb de pBU3. L'ADN de pBU3 est digéré avec SalI, les fragments religaturés, et utilisés pour transformer la souche QB254. 8 clones Cm$^R$ Lvs$^+$ ont été choisis et leurs plasmides ont été extraits. Un de ces plasmides recombinants, pBU14, qui contient un fragment SalI unique de 3,9 kb en plus du fragment SalI de 8 kb du vecteur a été plus particulièrement étudié. Ce fragment SalI de 3,9 kb comprend un fragment SalI-BamHI de 0,28 kb de pBR322 et un fragment SalI-BamHI de 3,6 kb (figure 2). Après une seconde étape de sous-clonage, le plasmide pBU16 a été isolé et il contient un fragment SalI-SphI d'environ 2,55 kb de l'ADN de B.subtilis. Ce plasmide restaure également l'activité de synthèse de la lévane-saccharase dans la souche mutante SacU$^-$QB254. De plus l'introduction soit de PBU14, soit de pBU16 dans une souche Rec$^-$ 1A510 conduit à l'hyperproduction de lévane-saccharase (tableau I). Ces données montrent que le gène sacU, ou au moins une partie fonctionnelle de ce gène, est localisé à l'intérieur du fragment SalI -SphI de 2,55 kb.

### TABLEAU I

Secrétion de lévane-saccharase par la souche 1A510 contenant les plasmides recombinants indiqués ci-dessus.

| Plasmide | Activité spécifique de lévane-saccharase (U/mg x 10³) | |
|---|---|---|
| | glycerol | saccharose |
| pHV1431d (vecteur de contrôle) | < 10 | 17 |
| pBU14 | < 10 | 330 |
| pBU16 | < 10 | 670 |

La souche de B. subtilis 1A510 contenant les plasmides sus-indiqués est cultivée sur milieu MMCH additionné de chloramphenicol et soit de 1 % de glycerol (en poids/volume) ("glycerol"), soit de 2 % de saccharose (en poids/volume) ("saccharose"). Les surnageants des cultures de cellules en phase exponentielle de croissance ont été dialysés pendant une nuit contre TPK (tampon phosphate de potassium) 0,05 M, pH 6,0, et les activité enzymatiques de la lévane-saccharase ont été mesurées. Les activités spécifiques ont été quantifiées par unités de lévane-saccharase par mg de protéine totale.

3) Techniques d'hybridation sur filtre de nitrocellulose

- purifier à partir du plasmide pBU16 le fragment SalI-SphI (2,55 kb),
- marquer ce fragment radioactivement par translation de césure (nick-translation),
- transférer l'ADN à tester sur un filtre de nitrocellulose,
- déposer ce filtre sur du papier Whatman n° 3 (marque déposée) imbibé de NaOH 0,5 N, NaCl 0,9 M et laisser 20 minutes à température ambiante,
- sécher brièvement sur papier,
- répéter l'opération sur papier imbibé de 1M Tris-HCl, pH 7, 10 minutes,
- sécher brièvement,
- répéter l'opération sur papier imbibé de 2 x SSC (1 x SSC = 0,15 M NaCl, 0,015 M Na₃citrate, pH 7),
- sécher sur papier, puis à l'étuve à 80°C pendant 2 heures,
Système homologue : poursuivre selon A (conditions stringentes),
Système hétérologue : poursuivre selon B (conditions non stringentes).

A) Conditions stringentes
- Hybrider le filtre avec 20 à 40 ml de tampon d'hybridation (50 % formamide, 5 x SSC, 1 x Denhardt*). Préhybrider pendant 2 heure à 42°C.

(* solution de Denhardt : 1 % Ficoll, 1 % polyvinylpyrrolidone, 1 % BSA.)
- Changer le tampon d'hybridation et ajouter environ 4 x 10⁶ c.p.m. de la sonde dénaturée (5 minutes à 100°C).
- Incuber de 16 à 24 heures à 42°C.
- laver successivement avec :
1 x 50 ml 5 x SSC, 50 % formamide 20 minutes à 42°C,
2 x 50 ml 2 x SSC, 0,1 % SDS 2 x 20 minutes à 42°C,
1 x 50 ml 0,2 x SSC, 0,1 % SDS 20 minutes à 42°C.

B) Conditions non stringentes
- Hybrider le filtre avec 20 à 40 ml de tampon d'hybridation (5 x SSC, 1 x Denhardt*, 0,1 % SDS), pré-hybrider pendant 1 heure à 42°C.
- Changer le tampon d'hybridation et ajouter environ 4 x10⁶ c.p.m. de la sonde dénaturée (5 minutes à 100°C).
- Incuber de 16 à 24 heures à 42°C.
- Laver successivement avec
1 x 50 ml 5 x SSC, 0,1 % SDS 20 minutes à 42°C,
2 x 50 ml 2 x SSC, 0,1 % SDS 2 x 20 minutes à 42°C.
(WAHL et al, Proc. Natl. Acad. Sci., USA :76, 3683-3687 (1979) ; MANIATIS et al, Molecular Cloning, Cold Spring Harbor Press, NEW YORK (1982).

4) ANALYSE GENETIQUE FINE DE LA STRUCTURE DU LOCUS sacU.

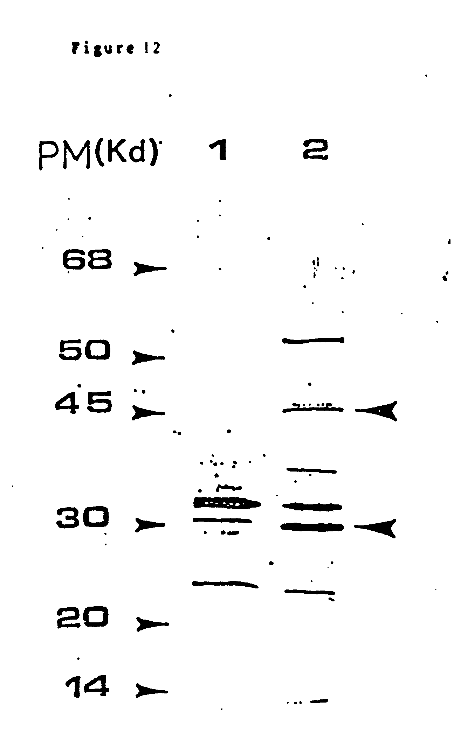

Afin de localiser le locus sacU à l'intérieur du fragment inséré dans pBU16, des délétions ont été effectuées au niveau dudit fragment et les plasmides suivants ont été construits. Le plasmide pBU101 dérive du plasmide pBU14 par élimination d'un fragment EcoRI de 2,4 kb. Le plasmide pBU100 a été construit en introduisant ce fragment EcoRI au niveau du site unique EcoRI du plasmide vecteur pHV1432. Puisque les deux plasmides pBU100 et pBU101 sont incapables de transformer la souche QB254 de génotype sacU⁻ en une souche de génotype sacU⁺ (Lvs⁺) (figure 12), il a été conclu que les séquences d'ADN essentielles entourent le site EcoRI de pBU16. Ceci a été confirmé en introduisant des cassettes d'ADN au niveau des sites EcoRI et BstBI de pBU16 conduisant respectivement aux plasmides pBU102 et pBU103. Puisque pBU14 et pBU16 ne pouvaient pas être maintenus chez E. coli, ces plasmides pBU102 et pBU103 ont été obtenus par clonage direct chez B. subtilis. Une séquence d'ADN de 4,5 kb contenant lacZ et erm a été insérée au niveau du site EcoRI unique de pBU16 afin de créer pBU102. Ce plasmide a été introduit dans des cellules compétentes de B. subtilis 168 par sélection des transformants EmR CmR. Des cellules EmR CmS sensibles apparaissent spontanément par récombinaison homologue (un mécanisme de double crossover conduisant à l'intégration de lacZ erm dans le chromosome). Le plasmide pBU103 a été construit de la même manière en insérant un fragment ClaI de 1,55 kb contenant un marqueur de résistance à la kanamycine aphA3 provenant de Streptococcus faecalis (TRIEU-CUOT P. et al, (1983), Nucleotide sequence of the Streptococcus faecalis plasmid gene encoding 3'5"-amino-glycoside phosphotransferase type III. Gene. 23 : 331-341) au niveau du site unique BstBI de pBU16. Comme il l'a été indiqué ci-dessus, après introduction de pBU103 dans B.subtilis 168, les cellules KmR CmS apparaissent par insertion du marqueur KmR à l'intérieur du locus sacU. Ces fragments lacZ erm et aphA3 ainsi insérés au niveau des sites EcoRI et BstBI respectivement par l'intermédiaire des plasmides pBU102 et pBU103 confèrent aux cellules de B. subtilis ainsi transformées un phénotype Lvs⁻.

5) SEQUENCE NUCLEOTIDIQUE DU LOCUS sacU

Le fragment SphI-SalI de 2,55 kb de pBU16 a été séquencé au niveau de ses deux brins par la méthode de terminaison de chaîne aux didésoxy nucléotides (SANGER F. et al, (1977) DNA sequencing chain terminating inhibitors, Proc. Natl. Acad. Sci. USA, 74 : 5463-5467). Il a ainsi été trouvé deux phases ouvertes de lecture ci-après désignées par ORF1 (385 codons) et ORF2 (229 codons) (figure 10). Ces phases ouvertes de lecture sont précédées par des sites de liaison aux ribosomes correspondant respectivement aux séquences GGAGGGA ($\Delta G = -78 kJ/mol$) et AAGGAGG ($\Delta G = -74 kJ/mol$). Aucun signal de terminaison de transcription n'a été trouvé entre ORF1 et ORF2. ORF1 et ORF2 codent pour des polypeptides possédant des poids molécululaires de 44.906 daltons et 25.833 daltons respectivemement.

6) EXPRESSION IN VITRO DES POLYPEPTIDES CODES PAR sacU

Le plasmide pBU16 contenant le locus sacU a été incubé in vitro en présence de E. coli. Les polypeptides ont été marqués par de la méthionine ³⁵S (activité spécifique supérieure à 39TBq/mmole) et séparés par SDS-PAGE et visualisés par autoradiographie (figure 12). La comparaison des polypeptides synthétisés par le plasmide recombinant avec ceux codés par le plasmide pHV1431d indique la présence de bandes nouvelles, deux d'entre elles ayant des poids moléculaires apparents en accord avec ceux des polypeptides déduits à partir des ORF1 et ORF2 (respectivement 45 000 daltons et 30 000 daltons).

7) COMPARAISON DES SEQUENCES EN ACIDES AMINES DES POLYPEPTIDES SacU$_{S1}$ et SacU$_{S2}$ AVEC CELLES DE PROTEINES DE REGULATION CONNUES

Une recherche informatique des homologies avec d'autres protéines indique que le polypeptide SacU$_{S1}$ codé par ORF1 présente une homologie avec les membres d'une classe de protéines "capteurs", c'est-à-dire avec CheA de S. typhimurium, CpxA de E. coli, VirA de A. tumefaciens, PhoR de E. coli, NtrB de K. pneumoniae (figure 7) (DRUMMOND M. et al, (1986), Sequence and domain relationships of ntrC and nifA from Klebsiella pneumoniae : homologies to other regulatory proteins, EMBO. J., 5 : 441-477 ; NIXON B.T. et al, (1986), two-component regulatory systems responsive to environmental stimuli share strongly conserved domains with the nitrogen-assimilation regulatory genes ntrB and ntrC, Proc.

Natl. Acad. Sci., USA, 83 : 7850-7854 ; RONSON C. W. et al., (1987), Conserved domains in bacterial regulatatory proteins that respond to environmental stimuli, Cell, 49 : 579-581). Dans les parties C-terminales de ces protéines, 5 régions d'amino-acides conservées, qui ont été numérotées de 1 à 5 (du côté N-terminal au côté C-terminal), ont déjà été décrites (Stock A. et al, (1988), CheA protein, a central regulator of bacterial chemotaxis, belongs to a family of proteins that control gene expression in response to changing environmental conditions, Proc. Natl. Acad. Sci., USA, 85 : 1403-1407.

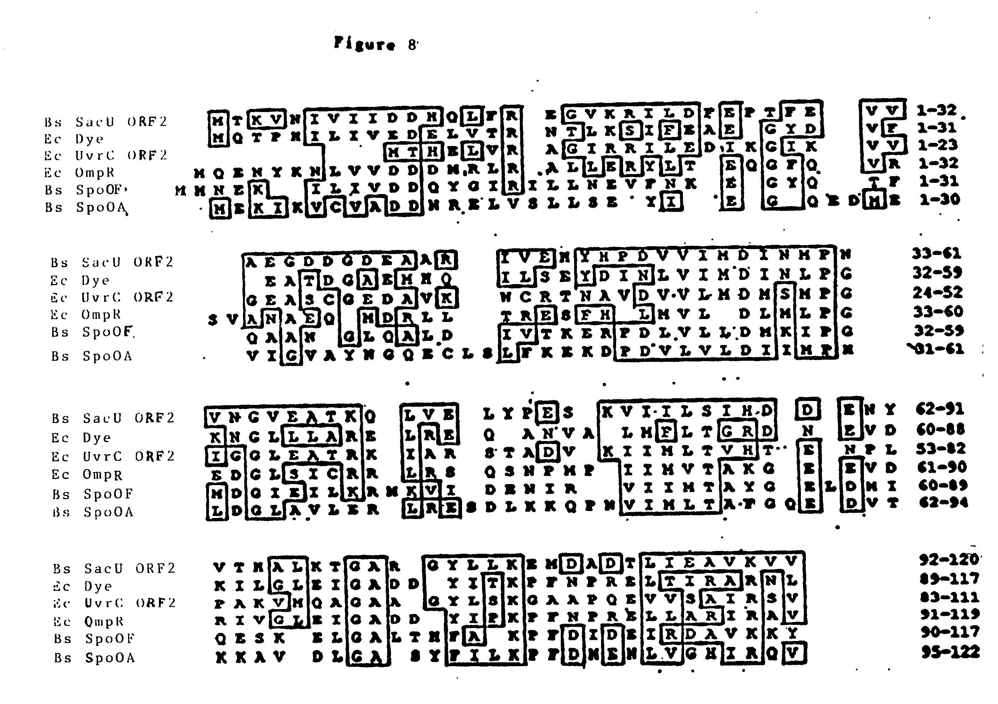

La protéine SacU$_{S2}$ codée par ORF2 présente des homologies avec une classe de protéines régulatrices (ou polypeptides "activateurs"). La protéine OmpR et Dye de E. coli et les protéines Spo0A et spo0F de B.subtilis présentent des homologies avec la protéine ORF2 du locus sacU au niveau N-terminal (figure 8). La protéine MalT de E. coli (Cole S.T. et al, (1986),The nucleotide sequence of the malT gene encoding the positive regulator of the Escherichia coli maltose regulation, Gene, 42 : 201-208) et le régulateur codé par ORF1 du locus uvrC (SHARMA S. et al, (1986), Multiple control elements for the uvrC gene unit of Escherichia coli, Nucleic Acids Res., 14 : 2301-2318) présentent des homologies avec la protéine codée par ORF2 du locus sacU au niveau C-terminal (figure 9) L'ORF2 du locus uvrC (référence identique à celle sus-mentionnée) est un cas spécial, puisqu'il présente des homologies avec le polypeptide codé par ORF2 au niveau des parties C- et N-terminales (figures 8 et 9).

Beaucoup des protéines sus-mentionnées fonctionnent par paires afin de réguler l'expression d'un gène au niveau de la transcription et ceci en réponse à un des changements de l'environnement tels que la limitation en éléments nutritifs ou une osmolarité altérée (MATSUYAMA, S. I. et al, (1986) Interaction between two regulatory proteins expression in ompF et ompC genes in Escherichia coli : a novel ompR mutation suppresses pleiotropic defects caused by an envZ mutation; J. Bacteriol., 168 : 1309-1314). Il pourrait en être de même pour les polypeptides SacU$_{S1}$ et SacU$_{S2}$ respectivement codés par ORF1 et ORF2 du locus sacU. La région N-terminale du polypeptide SacU$_{S2}$ peut soit interagir avec la protéine capteur ou avec l'appareil transcriptionnel lui conférant ainsi une fonction de régulateur positif ou d'activation de la transcription. Alternativemement, cette fonction peut se localiser au niveau C-terminal puisque ORF2 présente des homologies avec des régulateurs tels que MalT de E. coli.

L'interaction entre protéines capteur et régulateur conduit à une phosphorylation de l'effecteur (appelé également activateur ou régulateur) dans le cas des couples CheA/CheY, NtrB/NtrC et EnvZ/OmpR (NINFA and MAGASANIK et al, (1986), Covalent modification of the glnG product, NRI, by the gnlL product, NRII, regulates the transcription of the gnlALG operon Escherichia coli, Procl. Natl. Acad. Sci., USA,83, 5909-5913 ; WYLIE M. E. et al, (1988) Sensory transduction in bacterial chemotaxis involves phosphotransfer between Che proteins, Biochem. Biophys. Res. Comm., 151 : 891-896 ;

M.M. IG0 et T.J. SILHAVY (1988) "EnvZ, a transmembrane environmental sensor of Escherichia coli K-12 is phosphorylated in vitro, 170 : 5971-5973)). Il a été suggéré que la protéine capteur CheA subit une auto-phosphorylation en présence d'ATP afin de produire une phospho-CheA (J.F. HESS, R.B. BOURRET, M.I. SIMON (1988), "histidine phosphorylation and phosphoryl group transfer in bacterial chemotaxis", Nature, 336 : 139-143). La protéine capteur phosphorylée donne alors son groupe phosphoryl à l'activateur CheY d'une manière qui peut être comparable aux réactions de phosphotransférase du système de phosphotransférase des sucres (WYLIE D. et al, sus-mentionné). Pour ce qui concerne l'organisation du locus sacU, ORF1 et ORF2 peuvent appartenir à un même opéron, comme il l'a été proposé dans le cas d'autres systèmes de capteurs/activateurs (NtrB, NtrC) (NIXON B. T. et al, (1986), précédemment cité).

Le système sacU affecte la production d'enzymes qui dégradent les sources d'azote et de carbone (par exemple lévane-saccharase, α-amylase, β-glucanase(s), protéases). Il est probable que le signal transmis à la protéine capteur soit le reflet d'une limitation de carbone et/ou d'azote. Aucun segment transmembranaire n'a pu être détecté chez l'un ou l'autre des deux polypeptides codés par le locus sacU. Il est par conséquent probable que le système sacU réponde à un signal intracellulaire de limitation de matières nutritives.

8) Les propriétés du fragment d'ADN de 14 kb comportant le locus sacU$^h$ sus-mentionné sont plus particulièrement détaillées dans ce qui suit.

Une souche de B. subtilis QB4181 a été construite, portant à la fois la mutation sacU⁻42 et une fusion de transcription sacR-aphA3 (intégrée dans le chromosome de B. subtilis par double "crossing over" au locus sacC, le gène de structure de la lévanase). Le gène de résistance à la kanamycine aphA-3 est contrôlée par les signaux de transcription de la région sacR. Cette région contient notamment le promoteur du gène de structure sacB de la lévane-saccharase, la cible du gène régulateur sacU activant l'expression de sacB et la cible du gène régulateur sacS impliqué dans l'induction de la lévane-saccharase par le saccharose.

En raison de la présence de l'allèle sacU⁻42 dans la souche QB4081, l'expression de sacB et celle de sacR-aphA3 est déficiente. Par conséquent, la souche QB4181 est incapable de croître en présence de kanamycine sur boîte de milieu SMMHCKm100 (milieu minéral de Spizizen (J. Bacteriol.,81, 741-756 (1981)) additionné de saccharose (20 g/l), d'hydrolysat de caséine (0,5 g/l), de tryptophane (20 μg/ml), de kanamycine (100 μg/ml). Par contre, le remplacement de l'allèle sacU⁻42 par l'allèle sacU$^h$32 dans la souche QB4081 permet une activation de l'expression de sacR-aphA3 entraînant une résistance à la kanamycine.

Dans des expériences de transformation, mettant en jeu la souche QB4181 comme souche réceptrice et l'ADN chromosomique extrait d'un mutant sacU$^h$32 comme souche donneuse, des transformants (cellules transformées) sacU$^h$32 sont sélectionnés directement grâce à leur résistance à la kanamycine (Km^R) sur milieu SMMHCKm100.

D'une manière comparable, des transformants Km^R dans des expériences de transformation de la souche QB4181 à l'aide du plasmide pBUH1 ont été obtenus. Ces transformants portent l'allèle sacU^h32, car ils expriment complètement le phénotype "hyperproduction d'enzymes sécrétées". Un explication possible est que la méthode utilisée conduit à la sélection de recombinants dans lesquels la mutation sacU^h32 a pu être transférée du plasmide sur le chromosome. Ceci impliquerait que le plasmide utilisé comme ADN donneur porte la mutation sacU^h32 et que la présence d'un allèle chromosomique sacU^h32 entraîne l'expression complète du phénotype "hyperproduction d'enzymes secrétées".

La souche QB254 ou une souche Rec⁻ (1A510) porteuses du plasmide pBUH1 semblent produire, d'après des tests effectués sur boîtes de Petri, moins de protéases et moins de lévane-saccharase que la souche QB136 portant une mutation chromosomique sacU^h32. Puisque le fragment d'ADN couvrant la mutation sacU^h32 a bien été inséré dans le plasmide pBUH1 (cf. ci-dessus), il semble que, dans les conditions expérimentales utilisées, la mutation sacU^h32 portée par ce plasmide ne conduit qu'à une expression partielle du phénotype d'hyperproduction d'enzymes secrétées.

Il va de soi que toutes les techniques qui ont été décrites plus haut pour la transformation de cellules réceptrices, notamment des Bacillacées, avec le fragment de 2,55 kb, sont applicables au fragment de 14 kb ou des sous-fragments qui peuvent en être dérivés pour tirer pleinement profit des propriétés du locus sacU^h qu'ils contiennent, bien entendu sous réserve que les techniques soient adaptées en conséquence. Ces adaptations qui tiennent essentiellement aux différences de structures nucléotidiques des fragments de 2,55 kb et de 14 kb relèvent bien entendu aussi des compétences normales de l'homme du métier.

Les légendes correspondant aux figures illustrant les expériences et observations qui précèdent sont les suivantes :

- Figure 1 :
Carte de restriction du plasmide pBU3. La partie supérieure de la figure représente le fragment BamHI de l'ADN de B. subtilis BD1238. Les localisations du gène lacZ du marqueur de résistance à l'érythromycine (erm), et de Tn917 sont respectivement représentées par une bande hachurée, une bande noire et une autre bande hachurée. Le fragment SalI-SphI qui contient le locus sacU est indiqué par une bande blanche. La partie inférieure de la figure représente le vecteur plasmidique avec son origine de réplication chez B. subtilis (bande noire), le marqueur de résistance au chloramphénicol (Cm), et les séquences de pBR322 (bande blanche).

- Figure 2 :
Carte de restriction du plasmide pBU14. La partie supérieure de la figure représente le fragment SalI qui comprend le fragment d'ADN SalI-BamHI de 3,6 kb de B. subtilis (en pointillé) et un fragment BamHI-SalI de 0,28 kb de pBR322 (simple trait noir). Le plasmide pBU16, dérivé de pBU14 ne contient qu'un fragment de B.subtilis de 2,55 kb compris entre les sites SalI-SphI et indiqué par une bande blanche. La partie inférieure de la figure représente le vecteur plasmidique (symbôles identiques à ceux de la figure 1).

- Figure 3 :
Représentation schématique du plasmide pHV1436 (8,7 kb), se répliquant à un faible nombre de copies, et du plasmide pHV1431 (10,8 kb) se répliquant à un nombre élevé de copies. Les origines de réplication fonctionnelles chez B. subtilis, sont indiquées par une bande pointillée (pHV1436) ou par une bande noire (pHV1431) ; le marqueur de résistance au chloramphenicol est représenté par une bande hachurée, et les séquences de pBR322 sont indiquées par une bande blanche.

- Figure 4 :
Séquence nucléotidique et carte de restriction détaillées du fragment d'ADN comprenant le locus sacU de B.subtilis (la numérotation des nucléotides est décalée d'un nucléotide par rapport à la numérotation utilisée pour la figure 10 ; par exemple le nucléotide T en position 10 de la figure 10 est en position 11 sur la figure 4).

- Figure 5 :
Représentation de la phase de lecture ouverte de la séquence de 385 acides aminés du polypeptide SacU_{S1}.

- Figure 6 :
Repésentation de la séquence de 229 acides aminés du polypeptide SacU_{S2}.

- Figure 7 :
Comparaison des parties C-terminales du polypeptide SacU_{S1} codé par ORF1 du locus sacU de B. subtilis, et des protéines CheA de S. thyphimurium et CpxA de E. coli.

- Figure 8 :
Comparaison des parties N-terminales du polypeptide SacU_{S2} codé par ORF2 du locus sacU de B. subtilis, et des protéines Dye; UvrC (codé par l'ORF2), OmpR de E. coli, et SpoOA, SpoOF de B. subtilis.

- Figure 9 :
Comparaison des parties C-terminales du polypeptide SacU_{S2} de B. subtilis, et des protéines UvrC (codées par ORF1 et ORF2) et MalT de E. coli.

- Figure 10 :
Séquence nucléotidique détaillée du fragment d'ADN contenant le locus sacU selon l'invention. Les polypeptides SacU_{S1} et SacU_{S2} sont représentés au dessus des phases de lectures ouvertes ORF1 et ORF2 codant respectivement pour ces polypeptides ; ORF1 est délimitée par les nucléotides situés aux positions 220 et 1374, et ORF2 est délimitée par les nucléotides situés aux positions 1460 et 2146.

- Figure 11 :

Carte de restriction simplifiée d'insertion d'ADN dans des plasmides. La capacité des plasmides pBU14, pBU16, pBU100, pBU101, pBU102 et pBU103, à restaurer la synthèse de lévane-saccharase chez la souche de B. subtilis QB254 de génotype sacU⁻ est indiquée. Les positions de ORF1 et OFR2 sont indiquées par des bandes hachurées. La direction de la transcription est de gauche à droite. ORF2 est interrompue dans le plasmide pBU102 par une cassette lacZ erm, et ORF1 est interrompue dans le plasmide pBU103 par une cassette aphA3.

- Figure 12 :

Identification des polypeptides SacU$_{S1}$ et SacU$_{S2}$ : 1 μg d'ADN plasmidique circulaire, covalent et fermé a été utilisé dans cette manipulation in vitro. Les produits de traduction ont été marqués avec $^{35}$S méthionine, séparés par SDS-PAGE, et détectés par autoradiographie. La ligne 1 correspond au vecteur pHV1431d, et la ligne 2 au plasmide pBU16 contenant ORF1 et ORF2. La migration de protéines standard est indiquée par les flèches à gauche. Les flèches de droite indiquent des bandes correspondant à des poids moléculaires d'environ 45 000 et 30 000.

- Figure 13 :

Séquence d'acides aminés de la protéine DegS (sacU$_{S1}$)

Les protéines modifiés synthétisées dans les mutants hyperproducteurs ou dans les mutants déficients dans la production des enzymes dégradatives sont indiquées par des flèches vers le haut (↑) ou vers le bas (↓), respectivement. Exemple : la mutation degS200 (Hy) conduit à une hyperproduction d'enzymes dégradatives suite au remplacement à la position 218 de l'acide aminé G par l'acide aminé E dans la protéine DegU200.

Le domaine C-terminal qui présente des similarités avec NtrB, CheA et EnvZ est souligné.

Sont ainsi représentées les protéines DegS220, DegS200 et DegS100 conduisant respectivement aux génétypes degS220, degS200 et DegS100.

- Figure 14 :

Séquence d'acides aminés de la protéine DegU et les protéines modifiées synthétisées par des mutants degU (voir légende figure 13).

Sont ainsi représentées les protéines DegU32, DegU24, DegU146, DegU9, DegU31, DegU143, DegU200, DegU122 et DegU193, conduisant respectivement aux génotypes soulignés correspondant sur la figure.

- Figure 15 :

Mutations dans les gènes DegS (sacUS1) et DegU (sacUS2) et modifications dans les protéines correspondantes.

Les mutations degU24, degU500, degU31 et degU32 conduisent à une hyperproduction d'enzymes dégradatives; les autres mutations indiquées conduisent àà une déficience dans la production de ces enzymes. Les souches comme QB260, 261, 264, 266 et 269 portent deux mutations : la mutation degU32 (Hy) et une deuxième mutation, qui réverse l'effet de la mutation degU32, entraînant une déficience dans la production d'enzymes dégradatives.

Les souches de B. subtilis de génotypes sacU⁺ et sacUʰ utilisées pour le clonage des fragments d'ADN selon l'invention, ont été déposées à la COLLECTION NATIONALE DE CULTURES DE MICRO-ORGANISMES (C.N.C.M.) à l'INSTITUT PASTEUR de PARIS respectivement sous les numéros I-741 et I-744.

Une souche de B. subtilis contenant le plasmide pBUH1 a été déposée à la Collection Nationale des Cultures de Microorganismes (CNCM) de l'Institut Pasteur de Paris, France, le 13 juillet 1988 sous le n° I-782.

Il va sans dire que les dépôts de plasmides qui ont été effectués dans le cadre de la présente demande de brevet constituant des équivalents complets de la description des séquences nucléotidiques dont ils sont constitués, du seul fait qu'ils seront rendus accessible aux tiers dans les conditions prévus par le traité de Budapest et, de ce fait, séquençable par ces tiers.

**Revendications**

1. Fragment d'acide nucléique contenant une séquence fonctionnelle de nucléotides contenue dans un fragment SalI-SphI de 2,55kb, issu du chromosome d'une souche de B. subtilis ne présentant pas de mutation, ou d'une souche de B. subtilis présentant une mutation, notamment la mutation sacUʰ, ladite séquence étant caractérisée en ce qu'elle est capable, lorsqu'elle est introduite dans un B. subtilis- ou dans un autre micro-organisme, capable d'exploiter à son profit l'information portée à ce fragment- d'y induire une surproduction de protéines ou de lui conférer un phénotype Degʰ.

2. Fragment selon la revendication 1, caractérisé en ce que la séquence fonctionnelle est contenue dans un fragment de 2,55 kb issu du chromosome d'une souche de B. subtilis ne présentant pas de mutation sacuʰ, et en ce que ladite séquence porte le locus sacU codant pour le génotype sacU⁺.

3. Fragment selon la revendication 2, caractérisé en ce qu'il est compris dans la séquence nucléotidique représentée sur la figure 10.

4. Fragment selon la revendication 2 ou la revendication 3, caractérisé en ce que la séquence nucléotidique délimitée par les nucléotides situés aux positions 220 et 1374 de la figure 10 code pour le polypeptide SacU$_{S1}$, et en ce que la séquence nucléotidique délimitée par les nucléotides situés aux positions 1460 et 2146 de la figure 10 code pour le polypeptide SacU$_{S2}$.

5. Fragment selon l'une quelconque des revendications 2 à 4, caractérisé par des substitutions de certains de ses nucléotides par d'autres, sans que soit pour autant modifiée l'activité des polypeptides SacU$_{S1}$ et SacU$_{S2}$ qui lui correspondent.

6. Fragment d'acide nucléique caractérisé en ce qu'il est capable de s'hybrider avec le fragment Sall-SphI de 2,55 kb caractérisé par la séquence de nucléotides de la figure 10, et ce lorsque les opérations d'hybridation comprennent les étapes suivantes :
- pré-traitement (pré-hybridation) du filtre de nitrocellulose supportant ledit fragment d'acide nucléique avec le tampon d'hybridation (5 x SSC, 1 x Denhardt, 0,1 % SDS), cette opération étant effectuée à 42°C pendant 1 heure ;
- remplacement du tampon d'hybridation au contact du support, sur lequel le fragment d'acide nucléique est alors fixé, par du tampon d'hybridation de même composition, et addition de la séquence de 2,55 kb en tant que sonde, notamment marquée radioactivement, et préalablement dénaturée par un traitement à 100°C pendant 5 minutes ;
- incubation dudit fragment d'acide nucléique fixé sur le support dans ce tampon d'incubation avec le fragment de 2,55 kb à 42°C pendant une durée de 16 à 24 heures,
- l'élimination du tampon contenant la sonde non fixée,
. soit par lavages successifs avec 1 x 50 ml de 5 x SSC , 0,1 % SDS à 42°C pendant 20 minutes et 2 x 50 ml de 2 x SSC, 0,1 % SDS pendant 2 x 20 minutes à 42°C,dans des conditions non stringentes,
. soit par lavages successifs avec les solutions suivantes :
1 x 50 ml, 5 x SSC, 50 % formamide, 20 minutes à 42°C
2 x 50 ml, 2 x SSC, 0,1 % SDS, 2 x 20 minutes à 42°C
1 x 50 ml, 0,2 x SSC, 0,1 % SDS, 20 minutes à 42°C, dans des conditions stringentes.

7. Fragment d'acide nucléique selon la revendication 1 concernant une séquence fonctionnelle de nucléotides contenue dans un fragment d'ADN de 2,55 kb issu d'une souche de B. subtilis portant une mutation sacU$^h$, ladite séquence étant caractérisée en ce que :
- elle porte le locus sacU$^h$, codant pour le génotype sacU$^h$.
- elle est capable de restaurer l'activité de synthèse de lévane-saccharase chez une souche de B. subtilis portant une mutation sacU$^-$, lorsque ce fragment a été incorporé à cette souche,
- elle est capable d'induire une surproduction de protéine, ou de conférer un phénotype Deg$^h$, chez B. subtilis- ou chez un autre micro-organisme, capable d'exploiter à son profit l'information portée par ce fragment- lorsque ladite séquence est introduite dans ce micro-organisme
- elle est capable de s'hybrider-- et de rester hybridée-- avec un fragment d'ADN Sall-SphI de 2,55 kb issu de B. subtilis selon la revendication 1 dans les conditions décrites dans la revendication 6.

8. Fragment selon l'une quelconque des revendications 1 à 7 caractérisé en ce qu'il comporte une séquence codant pour au moins un polypeptide conduisant au phénotype Deg$^h$, notamment le polypeptide SacU$_{S1}$ et/ou SacU$_{S2}$ et/ou SacU$_{S}$$^h$, associée avec un promoteur sous le contrôle duquel la transcription de ladite séquence est susceptible d'être effectuée et, le cas échéant, une séquence codant pour des signaux de terminaison de la transcription.

9. Fragment selon la revendication 8, caractérisé en ce que le promoteur et, le cas échéant, la séquence codant pour les signaux de terminaison de la transcription sont hétérologues vis-à-vis de la séquence codant pour le (ou les) polypeptide conduisant au phénotype Deg$^h$.

10. Vecteur recombinant, notamment plasmide recombinant, du type réplicatif ou intégratif, capable de transformer une souche d'un micro-organisme approprié, notamment de B. subtilis, ledit vecteur contenant un fragment selon l'une quelconque des revendications 1 à 9 sous le contrôle d'un promoteur reconnu par les polymérases de ladite souche de micro-organisme.

11. Procédé visant à accroître la capacité de production d'enzymes, de protéines ou de polypeptides, par un micro-organisme approprié, ce procédé comprenant la transformation des cellules dudit micro-organisme au moyen d'un fragment selon l'une quelconque des revendications 1 à 9 ou d'un vecteur, notamment un plasmide selon la revendication 10, la mise en culture des cellules hôtes transformées dans un milieu de culture approprié et la récupération desdites protéines à partir de ces cellules ou du milieu de culture.

12. Procédé selon la revendication 11, caractérisé en ce que le micro-organisme approprié est une souche de Bacillus, notamment de B. subtilis, et en ce que les protéines dont la production est stimulée sont celles codées par les gènes de structure naturels de ladite souche de Bacillus activables par le gène sacU et/ou sacU$^h$.

13. Procédé selon la revendication 1, caractérisé en ce que le génome, ou le chromosome, des cellules de la souche de micro-organisme utilisée, a été modifié de manière à contenir une séquence nucléotidique codant pour un polypeptide déterminé dont la production doit être stimulée, cette séquence étant elle-même précédée d'une séquence cible pour le, ou les polypeptides codés par un fragment d'ADN selon les revendications 1 à 9.

14. Procédé selon la revendication 13, caractérisé en ce que la séquence cible contient celle qui est normalement comprise dans un fragment de 440 paires de bases, délimité par les sites de restriction Sau3A1-Rsa1, et situé en amont du site de liaison avec les ribosomes et de la séquence d'ADN codant pour la lévane-saccharase chez B. subtilis.

15. Procédé selon l'une quelconque des revendications 11 à 14, caractérisé en ce que le polypeptide dont la production doit être stimulée est une protéine secrétée, normalement synthétisée d'abord sous la forme d'un précurseur de cette protéine, et en ce que la séquence nucléotidique qui code pour cette protéine secrétée est précédée d'une séquence signal codant pour un peptide signal, dont le rôle est de permettre la secrétion dudit polypeptide, ladite séquence signal est elle-même précédée par un fragment de nucléotides comprenant une séquence cible.

16. Procédé selon l'une quelconque des revendications 11 à 15, caractérisé en ce que le génome, ou le chromosome des cellules du micro-organisme utilisé, a été modifié de manière à contenir une séquence nucléotidique codant pour un autre polypeptide, conduisant au phénotype Deg$^h$, que celui, ou ceux codés par un fragment d'ADN selon les revendications 1 à 9.

17. Procédé selon la revendication 16, caractérisé en ce que l'activateur autre que celui ou ceux codés par un fragment d'ADN des revendications 1 à 11, est le polypeptide SacQ$_L$.

18. Souche de micro-organisme, notamment de B. subtilis, caractérisée par le fait qu'elle est transformée par un fragment selon l'une quelconque des revendications 1 à 9ou par le vecteur selon la revendication 11.

19. Souche bactérienne selon la revendication 18, caractérisée en ce qu'elle est transformée par le fragment d'ADN comprenant une séquence d'ADN codant pour un polypeptide déterminé précédée par une séquence nucléotidique contenant une séquence cible et les éléments nécessaires pour permettre l'expression de la séquence codant pour la synthèse endocellulaire du polypeptide déterminé.

20. Souche bactérienne selon la revendication 19, caractérisée en ce que la séquence codant pour le polypeptide déterminé est précédée par une séquence signal qui est elle-même précédée par une séquence nucléotidique contenant une séquence cible et les éléments nécessaires pour permettre l'expression de la séquence codant pour la synthèse exocellulaire du polypeptide déterminé.

21. Polypeptide SacU$_{S1}$ caractérisé par la structure en acides aminés représentée sur la figure 5.

22. Polypeptide SacU$_{S2}$ caractérisé par la structure en acides aminés représentée sur la figure 6.

Figure 1

EP 0 349 353 A1

Figure 2

Figure 3

```
         10         20         30         40         50         60         70         80         90        100
GCATGCTAGCTGACCCTCCTGCTAAGCATAAAAGACTGCCTATACAAATTCGTACAGTCTTTAGAATTTTTGAGGTATTTTGGTATCATAAAGAGTAAGA
^^   ^^ ^        ^       ^^                            ^                        ^

SPHI MAEI      MNLI  ESPI                              RSAI                      MNLI
NLAIII              DDEI
   NHEI
     ALUI


        110        120        130        140        150        160        170        180        190        200
TAGTATATAAAAATGTTTTTTCTAGATATACGCATCTTCATTATAATTCGACATAATTTGCAGATCAATTACATTTAATAATAAAAATATATGACAACGC
                ^^            ^  ^          ^          ^                ^

                XBAI         SFANI         TAQI        DPNI
                MAEI         MBOII         TTHHB8      MBOI
                                                      NDEII
                                                      SAUIIIA


        210        220        230        240        250        260        270        280        290        300
CGTGACGGAGGGAAATTATGAATAAAACAAAGATGGATTCCAAAGTGCTGGATTCTATTTTGATGAAGATGCTGAAAACCGTTGACGGGAGCAAGGCGAG
 ^      ^                          ^  ^          ^             ^  ^                ^                    ^

MAEIII                            HINFI          HINFI         MBOII             HINDII                MNLI
  MNLI                            BSTXI                        SFANI


        310        320        330        340        350        360        370        380        390        400
GTTTTTGAAATCGGGGAGCAGTCAGCGGAGCAGTATGAACAGCTGGTCGAAGAACTGAAACAAATTAAACAGCAGGTGTATGAAGTGATTGAGCTTGGCG
                                      ^^     ^  ^                                                  ^

                                    PVUII  TAQI                                                   ALUI
                                    ALUI   TTHHB8
                                           MBOII            Figure 4
```

EP 0 349 353 A1

EP 0 349 353 A1

```
      410        420        430        440        450        460        470        480        490        500
ATAAACTTGAAGTGCAAAGTCCAT GCGAGAAACCGTTTATCCGAGGTCAGCCGTAATTTT CATAGATTCAGTGAAGAGGAAATCGCAATGCTTATGAAAA
                     ^                         ^                      ^       ^ ^

              NLA I I I                  MNL I                     H I NF I   MBO I I
                                                                              MNL I


      510        520        530        540        550        560        570        580        590        600
AGCCCATAAGCTGCAGGTAGAATTGACGATGATCCAGCAGCGTGAGAAGCAATTGCGCGAACGGCCGGACGATTTGGAGCGCAGA TTGCTAGGGCTTCAGG
          ^^^                     ^       ^                   ^^                          ^            ^

          ALU I               DPN I    BBV I              CFO I                       CFO I       MAE I
          BBV I               MBO I    FNU4H I            HHA I                       HHA I
          FNU4H I             NDE I I                     HINP I                      HINP I
          PST I               SAU I I I A                 ACC I I
                                                          FNUD I I
                                                          THA I


     ·610        620        630        640        650        660        670        680        690        700
AAATCATTGAGC GGTCAGAATCATTAGTAAGCCAAATTACAGTTGTGCTCAACTACTTGAATCAGGATTTGCGCGAAGTTGGACTGCTTCTTGCTGATGC
          ^                               ^            ^         ^^                          ^   ^    -

          HINF I                      BSP1206      HINF I     CFO I                       SFAN I
                                      HGIA I                  HHA I
                                                             HINP I                       DDE I
                                                             ACC I I
                                   Figure 4                  FNUD I I
                                   (suite n°1)               THA I
```

EP 0 349 353 A1

```
        710        720        730        740        750        760        770        780        790        800
TCAGGCAAAACAGGATTTCGGCTTAAGAATTATTGAGGCGCAGGAAGAAGAGCCGAAAAAGAGTCTCAAGAGAAAATCCATGACGGACCCGCTCAAATGCT
                  ^                      ^   ^      ^   ^                        ^               ^   ^
             AFLII         MNLI      MBOII          HINFI              NLAIII
                           CFOI      MBOII                             AFL I
                           HHAI                                        AVAII
                           HINPI                                       CLA II
                                                                       SAU96A
                                                                       SINI

        810        820        830        840        850        860        870        880        890        900
GGCGAATGTTATGATGAGATCGGAATTAATCGAGCGGATTTTCGTGACCGGGGCGCAGAGGCGGATTCCAAGAAATTAAAAATCTCCGCCAAAATGTTCG
           ^              ^              ^   ^   ^   ^   ^
      DPNI        TAQI          MAEIII   CFOI MNLI HINFI
      MBOI        TTHHB8        HAPII
      NDEII                     HPAII
      SAUIIIA                   MSPI HHAI
                               NCII HINPI
                               SCRFI

        920        930        940        950        960        970        980        990       1000
GAATGCCCTTTACGAAGTGAGAAGGATTATATATGATTTAAGACCGATGGCCCTTGATGACCTAGGCCTGATTCCAACTTTAAGAAAATATCTATATACA
                                        ^       ^^ ^^      ^
                                    HAEIII      STYI       HINFI
                                    SAU96A      MAEI
                                                STUI
                                                AATI
                                                HAE III
```

Figure 4
(suite n°2)

ACCGAGGAATATAACGGGAAGGTCAAAATACATTTTCAGTGCATTGGAGAAACAGAGGATCAGAGGCTAGCGCCTCAGTTTGAGGTTGCGCTCTTCAGGC
1050     1060     1070     1080     1090     1100

MNLI

MNLI    MNLI   HAEII     MNLI  CFOI
DPNI    NHEI   MNLI       HHAI
MBOI    MAEI   DDEI       HINPI
NDEII       CFOI           MBOII
SAUIIIA    HHAI
HINPI

TCGCACAGGAAGCTGTGTCTAATGCGCTAAAGCATTCTGAATCTGAAGAAATTACAGTCAAAGTTGAGATCACAAAGGATTTTGTGATTTTAATGATAAA
1110   1120   1130   1140   1150   1160   1170   1180   1190   1200

ALUI      CFOI   BSMI   HINFI MBOII          DPNI
HHAI                        MBOI
HINPI                      NDEII
SAUIIIA

AGATAACGGTAAAGGGTTCGACCTGAAGGAAGCGAAAGAGAAGAAAAACAAATCATTCGGCTTGCTGGGCATGAAAGAAAGAGTAGATTTATTGGAAGGA
1210   1220   1230   1240   1250   1260   1270   1280   1290   1300

TAQI            MBOII                NLAIII
TTHHB8

Figure 4
(suite n°3)

EP 0 349 353 A1

ACGATGACAATAGATTCGAAAATAGGTCTTGGGACATTTATTATGATTAAGGTTCCGTTATCTCTTTGACTATGATTTGTAAAATAGAGCCAAAAGGCAT

1310 1320 1330 1340 1350 1360 1370 1380 1390 1400

HGAI

HINFI

ASUII

TAQI

TTHHB8

ATTGACCGAATGCTAGAGTATATAGAACAATAATACAACGAGGCGTGGCTTGTGACTAAAGTAAACATTGTTATTATCGACGACCATCAGTTATTTCGTG

1410 1420 1430 1440 1450 1460 1470 1480 1490 1500

BSMI MAEI MNLI MAEIII TAQI

TTHHB8

AAGGTGTTAAACGGATATTGGATTTTGAACCTACCTTTGAAGTGGTAGCCGAAGGTGATGACGGGGACGAAGCGGCTCGTATTGTTGAGCACTATCATCC

1510 1520 1530 1540 1550 1560 1570 1580 1590 1600

HPHI FNU4HI BSP 1286 FOKI

HGIAI

Figure 4
(suite n°4)

EP 0 349 353 A1

EP 0 349 353 A1

```
     1610      1620      1630      1640      1650      1660      1670      1680      1690      1700
TGATGTTGTGATCATGGATATCAATATGCCAAACGTAAATGGTGTGGAAGCTACAAAACA GCTTGT AGAGCTGTA TCCTGA ATCT AAAG TAATTATTCTA
    ^^  ^    ^        ^                              ^          ^         ^        ^
    BCLI    ECORV                                  ALU I      ALU I    ALU I    HINFI
     DPNI
     MBOI
     NDEII
     SAUIIIA
        NLAIII


     1710      1720      1730      1740      1750       1760      1770      1780      1790      1800
TCAATTCACGATGACGAAAATTATGTAACACATGCCCTGAAAACAGGTGCAAGAGGTTATCTGCTGAAAGAGATGGATGCTGATACATTAATTGAAGCGG
              ^        ^                        ^                        ^^
             MAEIII                            MNLI                     FOKI
                  NLAIII                                                SFANI


     1810      1820      18 30      1840      1850      1860      1870      1880      1890      1900
TTAAAGTAGTGGCTGAGGGCGGATCTTA CCTCCATCCGAAGGTTACTCACAACCTCGTTA ACGAATTCCGCCGCCTTGCA ACAAGCGGAGTTTCTGCACA
        ^ ^          ^^       ^    ^              ^             ^    ^       ^         ^
       DDEI      XHOII    MNLI         MAEIII     MNLI       ECORI  FNU4HI
         MNLI    DPNI         FOKI                       HIND II
                 MBOI                                    HPAI
                 NDEII                                              Figure 4
                 SAUIIIA                                            (suite n°5)
```

EP 0 349 353 A1

```
        1910      1920      1930      1940      1950      1960      1970      1980      1990      2000
CCCTCAACATGAGGTTTACCCTGAAATCCGCAGACCATTACATATTTTAACTAGGCGGGAATGTGAAGTGCTGCAGATGCTTGCAGACGGAAAAAGCAAC
    ^     ^  ^                                          ^                    ^^       ^ ^                 ^

   MNLI   NLAIII                                       MAEI                 BBVI   SFANI               SACII
          MNLI                                                             FNU4HI  TTH11111            SSTII
                                                                           PSTI
```

```
       2010      2020      2030      2040      2050      2060      2070      2080      2090      2100
CGCGGTATTGGTGAATCATTGTTTATCAGTGAGAAAACCGTTAAAAACCATGTCAGCAATATTTTACAAAAAATGAATGTAAACGACCGGACGCAAGCCG
^          ^  ^                           ^ ,        ^                                 ^ ^

ACCII      HPHI                          NLAIII    SSPI                              HAPII
FNUDII     HINFI                                                                     HAPII
THAI                                                                                MSPI
                                                                                    HGAI
```

```
      2110      2120      2130      2140      2150      2160      2170      2180      2190      2200
TTGTGGTCGCCATTAAAAATGGCTGGGTAGAAATGAGATAGTATAATAGGAGACTTGCCTTTTACTAGGCAGGTCTTTTTTTTAGGCTGCCGTTTCCCTTA
                                                            ^                    ^

                                                           MAEI                 BBVI
                                                                                FNU4HI
```

```
      2210      2220      2230      2240      2250      2260      2270      2280      2290      2300
CAATAGAGTTATAAAGCAATAAGGCAGGTATCGAAGCTATGAATATTGCAGTCGTAACAGACAGCACGGCATATATTCCGAAAGAAATGCCGTGAACAACA
                                 ^     ^     !          ^

                                TAQI   SSPI  MAEIII
                                TTHHB8
                                    ALUI
```

Figure 4
(suite n°6)

```
      2310      2320      2330      2340      2350      2360      2370      2380      2390      2400
TCAGATACATATGATCCCTCTCCAGGTTGTTTTTAGGGAGGAGACTTACCGTGAAGAAATTGAGTTGGACTGGAAAAGCTTTTATGAAGAAGTGAAAAAA
    ^       ^    ^    ^                            ^                  ^                        ^^        ^
  NDEI  DPNI      APYI          MNLI            MBOII                              HINDIII    MBOII
        MBOI      BSTNI                                                            ALUI
        NDEII     ECORII
        SAUIIIA   SCRFI
          MNLI


      2410      2420      2430      2440      2450      2460      2470      2480      2490      2500
CATAATGAGCTCCCGACGACTTCTCAGGCCAATCGGCGAGCTGGTTGCGTTGTATGAAGAGCTTGGCAAGTCTTATGATGCGGTTATCAGTATCCATCTT
    ^^              ^    ^              ^              ^  ^                  ^
  BANII           DDEI          ALUI            MBOII            SFANI
  BSP1286            HAEIII                      ALUI
  HGIAI
  SACI
  SSTI
   ALUI


  2510      2520      2530      2540      2550
TCCAGCGGGATCAGCGGAACATTCAGCAGTGCAGCAGCGGCTGATTCGATGGTCGAC
    ^                        ^    ^    ^      ^   ^      ^^
   DPNI                   BBVI  FNU4HI   TAQI  ACCI
   MBOI                   FNU4HI       HINFI   HINDII
   NDEII                     BBVI        TTHHB8 TAQI
   SAUIIIA                 FNU4HI          SALI
                                          TTHHB8
```

Figure 4
(suite n°7)

MNKTKMDSKVLDSILMKMLKTVDGSKDEVFQIGEQSRQQYEQLVEELKQI    50

KQQVYEVIELGDKLEVQTRHARNRLSEVSRNFHRFSEEEIRNAYEKAHKL    100

QVELTMIQQREKQLRERRDDLERRLLGLQEIIERSESLVSQITVVLNYLN    150

QDLREVGLLLADAQAKQDFGLRIIEAQEEERKRVSREIHDGPAQMLANVM    200

MRSELIERIFRDRGAEDGFQEIKNLRQNVRNALYEVRRIIYDLRPMALDD    250

LGLIPTLRKYLYTTEEYNGKVKIHFQCIGETEDQRLAPQFEVALFRLAQE    300

AVSNALKHSESEEITVKVEITKDFVILMIKDNGKGFDLKEAKEKKNKSFG    350

LLGMKERVDLLEGTMTIDSKIGLGTFIMIKVPLSL    385

Polypeptide SacU$_{S1}$ (ou DegS)

Figure 5

EP 0 349 353 A1

<u>SEQUENCE D'ACIDES AMINES DEDUITE DE LA PHASE DE LECTURE OUVERTE</u> ORF2

50
MTKVNIVIIDDHQLFREGVKRILDFEPTFEVVAEGDDGDEAARIVEHYHPDVVIMDINMPNVNGVEATKQLVELYPESKVIILSIHDDENYV

100                                                                         150
THALKTGARGYLLKEMDADTLIEAVKVVAEGGSYLHPKVTHNLVNEFRRLATSGVSAHPQHEVYPEIRRPLHILTRRREGEVLQMLADGKSNRG

200                          229
IGESLFISEKTVKNHVSNILQKMNVNDRTQAVVVAIKNGHVEMR

Figure 6

EP 0 349 353 A1

Figure 7

```
                                                                            306-338
                                                                            448-480
Bs SacU ORF1   L K H S E S E E I T V K V E I T K D F V I L M I K D N G K G F D L   398-409
St CheA        L A K A M S Q G M A V N E N M T D D E V G M L I F A P G F S T A E
Ec CpxA        E Q                                        I F R P F Y R T D E
```

région 4

```
                                                                                  339-374
Bs SacU ORF1   K E A K E K K N K S F G L L G M K E R V D L L E G T M T I D S K I G L G   481-515
St CheA        Q V T D V   S G R G V G M D V V K R N I I Q E M G G H V V E I Q S K Q G S G   410-445
Ec CpxA        A R D R E S G G T G L G L A I V E T A I Q Q H R G W V K A E D S P L G G
```

région 5

```
Bs SacU ORF1   T F I M I K V P L S L   375-385
St CheA        T T I R I L L P L T L   516-526
Ec CpxA        L R L V I W L P L Y K R S   446-458
```

Figure 8

EP 0 349 353 A1

| | | | 1-32 |
|---|---|---|---|
| Bs SacU ORF2 | M T K V N I V I I D D H Q L F R | E G V K R I L D F E P T F E | V V | 1-32 |
| Ec Dye | M Q T F N I L I V E D E L V T | R N T L K S I F E A E | V F | 1-31 |
| Ec UvrC ORF2 | M T H E L V R | R A G I R R I L E D I K G I K | V V | 1-23 |
| Ec OmpR | M Q E N Y K N L V V D D D M R L R | R A L L E R Y L T E Q G F Q | V R | 1-32 |
| Bs SpoOF | M M N E K I L I V D D Q Y G I R | I L L N E V F N K E G Y Q | I F | 1-31 |
| Bs SpoOA | M E K I K V C V A D D N R E L V S L L S E Y I E G Q E D M H | | | 1-30 |

| | | | |
|---|---|---|---|
| Bs SacU ORF2 | A E G D D G D E A A R | I V E N Y H P D V V I M D I N M P N | 33-61 |
| Ec Dye | E A T D G A E M N Q | I L S E Y D I N L V I M D I N L P G | 32-59 |
| Ec UvrC ORF2 | G E A S C G E D A V K | W C R T N A V D V V L M D M S M P G | 24-52 |
| Ec OmpR | S V A N A E Q M D R L L | T R E S F H L M V L D L M L P G | 33-60 |
| Bs SpoOF | Q A A N G L Q A L D | I V T K E R P D L V L L D M K I P G | 32-59 |
| Bs SpoOA | V I G V A Y N G Q E C L S L F K E K D P D V L V L D I I M P N | | 31-61 |

| | | | |
|---|---|---|---|
| Bs SacU ORF2 | V N G V E A T K Q L V E L Y P E S K V I I L S I H D D E N Y | | 62-91 |
| Ec Dye | K N G L L L A R E L R E Q A N V A L M F L T G R D N E V D | | 60-88 |
| Ec UvrC ORF2 | I G G L E A T R K I A R S T A D V K I I M L T V H T N P L | | 53-82 |
| Ec OmpR | E D G L S I C R R L R S Q S N P M P I I M V T A K G E E V D | | 61-90 |
| Bs SpoOF | M D G I E I L K R M K V I D E N I R V I I M T A Y G E L D M I | | 60-89 |
| Bs SpoOA | L D G L A V L E R L R E S D L K K Q P M V I M L T A F G Q E D V T | | 62-94 |

| | | | 92-120 |
|---|---|---|---|
| Bs SacU ORF2 | V T N A L K T G A R G Y L L K E M D A D T L I E A V K V V | | 92-120 |
| Ec Dye | K I L G L E I G A D D Y I T K P F N P R E L T I R A R N L | | 89-117 |
| Ec UvrC ORF2 | P A K V M Q A G A A G Y L S K G A A P Q E V V S A I R S V | | 83-111 |
| Ec OmpR | R I V G L E I G A D D Y I P K P F N P R E L L A R I R A V | | 91-119 |
| Bs SpoOF | Q E S K E L G A L T H F A K P F D I D E I R D A V K K Y | | 90-117 |
| Bs SpoOA | K K A V D L G A S Y F I L K P F D M E N L V G H I R Q V | | 95-122 |

EP 0 349 353 A1

Figure 9

| | | | |
|---|---|---|---|
| Bs SacU ORF2 | L T R R E C E V L Q M L A D G K S N R G I G E S L F I S E K T | 166-196 |
| Ec UvrC ORF1 | F S K R E K E I L R W T A E G K T S A E I A M I L S I S E N T | 181-211 |
| Ec UvrC ORF2 | L S E R E L Q I M L M I T K G Q K V N E I S E Q L N L S P K T | 143-173 |
| Ec MalT | L T Q R E W Q V L G L I Y S G Y S N E Q I A G E L E V A A T T | 836-866 |

| | | | |
|---|---|---|---|
| Bs SacU ORF2 | V K N H V S N I L Q K M N V N D R T Q A V V V A I K N G W V E M R | 197-229 |
| Ec UvrC ORF1 | V N F H Q K N M Q K K I N A P N K T Q V A C Y A A A T G L I | 212-241 |
| Ec UvrC ORF2 | V N S Y R Y R M F S K L N I E G D V E L T H L A I R H G L C N A E | 174-206 |
| Ec MalT | I K T H I R N L Y Q K L G V A E R Q D A V Q H A Q Q L L K M M G | 857-898 |

EP 0 349 353 A1

CATGCTAGCTGACCCTCCTGCTAAGCATAAAAGACTGCCTATACAAATTCGTACAGTCTTTAGAATTTTTGTGCCGTAT

TTGGTATCATAAAGAGTAGATAGTATATAAAAATGTTTTTTTTCTAGAATATACGCATTCTTTCATTATAAT T CGACA
100

MetAsnLysThrLys
TAATTTGCAGATCAATTACATTTATAATAAAAATATATGACAACGCCGTGACGGAGGGAAATTATGAATAAAACAAAG
200

MetAspSerLysValLeuAspSerIleLeuMetLysMetLeuLysThrValAspGlySerLysAspGluValPheGln
ATGGATTCCAAAGTGCTGGATTCTATTTTGATGAAGATGCTGAAAACCGTTGACGGGAGCAAGGACGAGGTTTTTCAA
300

IleGlyGluGlnSerArgGlnGlnTyrGluGlnLeuValGluGluLeuLysGlnIleLysGlnGlnValTyrGluVal
ATCGGGGAGCAGTCACGCCAGCAGTATGAACAGCTGGTCGAAGAACTGAAACAAATTAAACAGCAGGTGTATGAAGTG

IleGluLeuGlyAspLysLeuGluValGlnThrArgHisAla ArgAsnArgLeuSerGluValSerArgAsnPheHis
ATTGAGCTTGGCGATAAACTTGAAGTGCAAACTCGCCATGCGAGAAACCGTTTATCCGAGGTCAGCCGTAAT T T TCAT
400

ArgPheSerGluGluGluIleArgAsnAlaTyrGluLysAlaHisLysLeuGlnValGluLeuThrMetIleGlnGln
AGATTCAGTGAAGAGGAAATCCGCAATGCTTATGAAAAAGCCCATAAGCTGCAGGTAGAATTGACGATGATCCAGCAG
500

ArgGluLysGlnLeuArgGluArgArgAspAspLeuGluArgArgLeuLeuGlyLeuGlnGluIleIleGluArgSer
CGTGAGAAGCAATTGCGCGAACGGCGGGACGATTTGGAGCGCAGATTGCTAGGGCTTCAGGAAATCATTGAGCGGTCA
600

GluSerLeuValSerGlnIleThrValValLeuAsnTyrLeuAsnGlnAspLeuArgGluValGlyLeuLeuLeuAla
GAATCATTAGTAAGCCAAATTACAGTTGTGCTCAACTACTTGAATCAGGATTTGCGCGAAGT TGG A C TG CTTCTTGCT
700

AspAlaGlnAlaLysGlnAspPheGlyLeuArgIleIleGluAlaGlnGluGluGluArgLysArgValSerArgGlu
GATGCTCAGGCAAAAACAGGATTTCGGCTTAAGAATTATTGAGGCGCAGGAAGAAGAGCGAAAAAGAGTCTCAAGAGAA

IleHisAspGlyProAlaGlnMetLeuAlaAsnValMetMetArgSerGluLeuIleGluArgIlePheArgAspArg
ATCCATGACGGACCCGCTCAAATGCTGGCGAATGTTATGATGAGATCGGAATTAATCGAGCGGAT TTTCCGTGACCGG
800

GlyAlaGluAspGlyPheGlnGluIleLysAsnLeuArgGlnAsnValArgAsnAlaLeuTyrGluValArgArgIle
GGCGCAGAGGACGGGATTCCAAGAAATTAAAAAATCTCCGCCAAAATGTTCGGAATGCCCTTTACGAAGTGAGAAGGATT
900

IleTyrAspLeuArgProMetAlaLeuAspAspLeuGlyLeuIleProThrLeuArgLysTyrLeuTyrThrThrGlu
ATATATGATTTAAGACCGATGGCCCTTGATGACCTAGGCCTGATTCCAACTTTAAGAAAATATCTATATACAAACCGAG
1000

GluTyrAsnGlyLysValLysIleHisPheGlnCysIleGlyGluThrGluAspGlnArgLeuAlaProGlnPheGlu
GAATATAACGGGAAGGTCAAAATACATTTTCAGTGCATTGGAGAAACAGAGGATCAGAGGCTAGCGCCTCAGTTTGAG

ValAlaLeuPheArgLeuAlaGlnGluAlaValSerAsnAlaLeuLysHisSerGluSerGluGluIleThrValLys
GTTGCGCTCTTCAGGCTCGCACAGGAAGCTGTGTCTAATGCGCTAAAGCATTCTGAATCTGAAGAAATTACAGTCAAA
1100

ValGluIleThrLysAspPheValIleLeuMetIleLysAspAsnGlyLysGlyPheAspLeuLysGluAlaLysGlu
GTTGAGATCACAAAGGATTTTGTGATTTTAATGATAAAAGATAACGGTAAAGGGTTCGACCTGAAGGAAGCGAAAGAG
1200

LysLysAsnLysSerPheGlyLeuLeuGlyMetLysGluArgValAspLeuLeuGluGlyThrMetThrIleAspSer
AAGAAAAACAAATCATTCGGCTTGCTGGGCATGAAAGAAAGAGTAGATTTATTGGAAGGAACGATGACAATAGATTCG
1300

LysIleGlyLeuGlyThrPheIleMetIleLysValProLeuSerLeu ***
AAAATAGGTCTTGGGACATTTATTATGATTAAGGTTCCGTTATCTCTTTGACTAT

Figure 10

GATTTGTAAAAATAGAGCCAAAAGGCATATTGACCGAATGCTAGAGTATATAGAACAATAATAC<u>AA GGAG GCG</u> TGGCTT

1400

MetThrLysValAsnIleValIleIleAspAspHisGlnLeuPheArgGluGlyValLysArgIleLeuAspPheGlu
GTGACTAAAGTAAACATTGTTATTATCGACGACCATCAGTTATTTCGTGAAGGTGTTAAAC GGA TA TTGGATT TTGAA

1500

ProThrPheGluValValAlaGluGlyAspAspGlyAspGluAlaAlaArgIleValGluHisTyrHisProAspVal
CCTACCTTTGAAGTGGTAGCCGAAGGTGATGACGGGGACGAAGCGGCTCGTATTGTTGAGCACTATCA TCC TGATGTT

1600

ValIleMetAspIleAsnMetProAsnValAsnGlyValGluAlaThrLysGlnLeuValGluLeuTyrProGluSer
GTGATCATGGATATCAATATGCCAAACGTAAATGGTGTGGAAGCTACAAAACAGCTTGTAGAGCTG TA TCCTGAATCT

LysValIleIleLeuSerIleHisAspAspGluAsnTyrValThrHisAlaLeuLysThrGlyAlaArgGlyTyrLeu
AAAGTAATTATTCTATCAATTCACGATGACGAAAATTATGTAACACATGCCCTGAAAACAGGTGCAAG A GG TTATCTG

1700

LeuLysGluMetAspAlaAspThrLeuIleGluAlaValLysValValAlaGluGlyGlySerTyrLeuHisProLys
CTGAAAGAGATGGATGCTGATACATTAATTGAAGCGGTTAAAGTAGTGGCTGAGGGCGGATCTTACCTC CATCC GAAG

1800

ValThrHisAsnLeuValAsnGluPheArgArgLeuAlaThrSerGlyValSerAlaHisProGlnHisGluValTyr
GTTACTCACAACCTCGTTAACGAATTCCGCCGCCTTGCAACAAGCGGAGTTTCTG CACACCCTCAACATGAGGT TTAC

1900

ProGluIleArgArgProLeuHisIleLeuThrArgArgGluCysGluValLeuGlnMetLeuAlaAspGlyLysSer
CCTGAAATCCGCAGACCATTACATATTTTAACTAGGCGGGAATGTGAAGTGCTGCAGATGCTTGCAGACGG A A A A AGC

2000

AsnArgGlyIleGlyGluSerLeuPheIleSerGluLysThrValLysAsnHisValSerAsnIleLeuGlnLysMet
AACCGCGGTATTGGTGAATCATTGTTTATCAGTGAGAAAACCGTTAAAAAACCATGTCAGCAATATTTTACAA A A A ATG

AsnValAsnAspArgThrGlnAlaValValValAlaIleLysAsnGlyTrpValGluMetArg***
AATGTAAACGACCGGACGCAAGCCGTTGTGGTCGCCATTAAAAAATGGCTGGGTAGAAATGAGATAGTATAATAG GAGA

>>>

2100

CTTGCCTTTTACTAGGCAGGTCTTTTTTTTAGGCTGCCGTTTCCCTTACAATAGAGTTATAAAGC AA TAAGGCAGGT AT
> >>>>>            <<<<< <<<<

2200

CGAAGCTATGAATATTGCAGTCGTAACAGACAGCACGGCATATATTCCGAAAGAAATGCGTGAACAACATCAGATACA

2300

TATGATCCCTCTCCAGGTTGTTTTTAGGGAGGAGACTTACCGTGAAGAAATTGAGTTGGACTGGAAAAGCT TTT ATG A

AGAAGTGAAAAAAACATAATGAGCTCCCGACGACTTCTCAGCCGCCAATCGGCGAGCTGGTTGCGTTGTATGAAGAGCT

2400

TGGCAAGTCTTATGATGCGGTTATCAGTATCCATCTTTCCAGCCGGGATCAGCGGAACATTCAGCAGTGCAGCAGC GGC

2500

TGATTCGATGGTCGAC

Figure 10 (suite)

EP 0 349 353 A1

Figure 11

PLASMIDE  ADN INSERE  COMPLEMENTATION
de sacU4H
(souche QB254)

ORF 1     ORF 2

pBU 14    SphI  Bam HI  Eco RI    SphI          Bst BI      Eco RI      Sal I      oui

pBU 16                            SphI          Bst BI      Eco RI      Sal I      oui

pBU 100            Eco RI                       Bst BI      Eco RI                  non

pBU 101    Bam HI   Eco RI                                  Eco RI      Sal I      non

pBU 102                           SphI              lac Z   erm          Sal I      non
                                                            Eco RI

pBU 103                           SphI         aph A3                    Sal I      non
                                               Bst BI       Eco RI

1 kb

Figure 12

EP 0 349 353 A1

MNKTKMDSKV LDSILMKMLK TVDGSKDEVF QIGEQSRQQY EQLVEELKQI

KQQVYEVIEL GDKLEVQTRH ARNRLSEVSR NFHRFSEEEI RNAYEKAHKL

QVELTMIQQR EKQLRERRDD LERRLLGLQE IIERSESLVS QITVVLNYLN

QDLREVGLLL ADAQAKQDFG LRIIEAQEEE RKRVSREIHD GPAQNLANVM

(degS200) E          (degS100) M      V (degS220)

MRSELIERIF RDRGAEDGFQ EIKNLRQMVR NALYEVRRII YDLRPMALDD

IGLIPTLRKY LYTEEYNGK VKINTOCIGE TEDQRLAPQF EVALFRLAQE

(degS39.degS42) K

AYSNALKHSE SEEITVKVEI TKDFVILMIK DNGKGFDLKK AKEKKNKSFG

LLGMKERVDL LEGTMTIDSK IGLGTFIMIK VPLSL*

Figure 13

(degU32)  L

MTKVNIVIID  DHQLFREGVK  RILDFEPTFE  VVAEGDDGDE  AARIVEHYHP

(degU24,degU500)  I

DVVIMDIKMP  NVNGVEATKQ  LVELYPESKV  IILSIHDDEN  YVTHALKTGA

N  (degU146)

K  (degU9,degU118)              L  (degU31)

RGYLLKEMDA  DTLIEAVKVV  AEGGSYLHPK  VTHNLVNEFR  RLATSGVSAH

PQHEVYPEIR  RPLHILTRRE  CKVLQMLADG  KSNRGIGESL  FISEKTVKHH

(degU143)  W         Q  (degU200)         C  (degU122)

VSNILQKMNV  NDRTQAVVVA  IKNGWVEMR*

(degU193)  V

Figure 14

## MUTATIONS degS/degU

| SOUCHE | GENOTYPE | CODON MODIFIE | | ACIDE AMINE MODIFIE | |
|---|---|---|---|---|---|
| QB315 | degU24(Hy) | ACA——ATA | 1752 | Thr——Ile | 98 |
| QB323 | degU500(Hy) | ACA——ATA | 1752 | Thr——Ile | 98 |
| QB152 | degU31(Hy) | GTT——CTT | 1850 | Val——Leu | 131 |
| QB254 | degS42 | GAA——AAA | 1117 | Glu——Lys | 300 |
| QB257 | degS39 | GAA——AAA | 1117 | Glu——Lys | 300 |
| QB256 | degU122 | CGC——TGC | 2009 | Arg——Cys | 184 |
| QB260 | degU32(Hy) degU143 | CGG——TGG | 1964 | Arg——Trp | 169 |
| QB261 | degU32(Hy) degU146 | GAT——AAT | 1625 | Asp——Asn | 56 |
| QB264 | degU32(Hy) degU193 | GCC——GTC | 2118 | Ala——Val | 220 |
| QB266 | degU32(Hy) degU200 | CGG——CAG | 1965 | Arg——Gln | 169 |
| QB269 | degU32(Hy) degS220 | GCC——GTT | 797 | Ala——Val | 193 |

Figure 15

EP 0 349 353 A1

**Office européen des brevets**

**RAPPORT DE RECHERCHE EUROPEENNE**

Numero de la demande

EP  89 40 0813

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,A | JOURNAL OF BACTERIOLOGY, vol. 161, no. 3, mars 1985, pages 1182-1187, American Society for Microbiology; E. AUBERT et al.: "Cloning and expression in Escherichia coli of the regulatory sacU gene from Bacillus subtilis" --- | | C 12 N  15/00<br>C 12 N   1/20<br>C 07 K  13/00 //<br>(C 12 N   1/20<br>C 12 R   1:125) |
| A | JOURNAL OF BACTERIOLOGY, vol. 146, no. 3, juin 1981, pags 1162-1165; D.M. WILLIAMS et al.: "Cloning restriction fragments that promote expression of a gene in Bacillus subtilis" --- | | |
| A | EP-A-0 117 823  (CNRS) --- | | |
| A | GENE, vol. 46, no. 2/3, 1986, pages 247-255, Elsevier Science Publishers B.V., Amsterdam, NL; M.M. ZUKOWSKI et al.: "Hyperproduction of an intracellular heterologous protein in a sacUh mutant of Bacillus subtilis" --- | | |
| A | CHEMICAL ABSTRACTS, vol. 102, no. 23, 10th June 1985, page 160, abstract no. 198916u, Columbus, Ohio, US; A. FOUET et al.: "The sucrose system as a model of genetic regulation in Bacillus subtilis", & GENET. BIOTECHNOL. BACILLI, [PROC. INT. CONF.], 2nd 1983, (Pub. 1984), 113-26 --- | | **DOMAINES TECHNIQUES RECHERCHES (Int. Cl.4)**<br><br>C 12 N |
| D,P<br>A | EP-A-0 261 009  (INSTITUT PASTEUR)<br><br>----- | | |

Le présent rapport a été établi pour toutes les revendications

| Lieu de la recherche | Date d'achèvement de la recherche | Examinateur |
|---|---|---|
| LA HAYE | 27-08-1989 | PULAZZINI A.F.R. |